# DEMANDE DE BREVET EUROPEEN

(11) **EP 0 651 982 A1**
(43) Date de publication de la demande: **10.05.1995**
(21) Numéro de dépôt: 94402506.3
(22) Date de dépôt: 07.11.1994
(51) Int. Cl.: A61F 9/00, B23K 26/06

(54) **Masque destiné à être interposé dans un faisceau laser afin d'appliquer un traitement déterminé, notamment d'ablation superficielle, à un organe ou à un objet, notamment une cornée, une lentille ou un implant cornéen; procédé et dispositif de traitement mettant en oeuvre un tel masque**

(30) Priorité: 08.11.1993 FR 9313244
(71) Demandeur: Hanna, Khalil, F-75007 Paris (FR)
(72) Inventeur: Hanna, Khalil, F-75007 Paris (FR)
(74) Mandataire: Martin, Jean-Jacques

(57) **Abrégé**

La présente invention concerne un masque destiné à être interposé dans un faisceau laser afin d'appliquer un traitement déterminé, notamment d'ablation superficielle, à un organe ou à un objet, notamment une cornée, une lentille ou un implant cornéen, ainsi qu'un procédé et un dispositif mettant en oeuvre un tel masque.

Ce masque (18) présente, outre une zone (44) opaque à la longueur d'onde du faisceau laser, une zone (43) transparente à cette longueur d'onde, dessinée de façon à former un ensemble fractal.

Ce masque (18) peut trouver notamment son application en chirurgie réfractive, pour corriger des myopies ou des hypermétropies avec ou sans astigmatisme ou pour effectuer des ablations de profondeur constante, de même qu'il peut être utilisé pour usiner superficiellement toute sorte d'objet, et notamment des lentilles optiques d'origine naturelle ou synthétique.

## Description

La présente invention concerne un masque destiné à être interposé dans un faisceau laser afin d'appliquer un traitement déterminé à un organe ou à un objet. Elle concerne également un procédé et un dispositif de traitement mettant en oeuvre un tel masque.

Elle s'intéresse plus particulièrement, mais non exclusivement, à l'utilisation d'un laser émettant dans le domaine des ultraviolets de courte longueur d'onde pour effectuer de la chirurgie cornéenne, afin de corriger des erreurs réfractives de l'oeil ou d'ôter des opacités cornéennes superficielles, par exemple, ou encore pour usiner une lentille destinée à être accolée à la cornée ou insérée dans celle-ci.

On appelle "chirurgie réfractive" la chirurgie destinée à corriger les erreurs réfractives de l'oeil. De nombreuses techniques ont été proposées à cet effet (WARING GO : "Making sens of Keratospeak IV. Classification of refractive surgery, Arch. Ophthalmol. 1992 ; Vol. 110:1385-1391"). Certaines de ces techniques utilisent le laser pour modifier la courbure de la surface antérieure de la cornée en prélevant de cette surface l'équivalent d'une lentille de puissance appropriée et de type positif ou négatif, et éventuellement cylindrique, selon qu'il s'agit de corriger une myopie ou une hypermétropie, et le cas échéant un astigmatisme. On parle alors de kératectomie photoréfractive ou kératomileusis au laser.

On peut également utiliser le laser pour oter des opacités cornéennes superficielles sans modifier la vergence de l'oeil, et l'on parle alors de kératectomie thérapeutique au laser.

Dans un cas comme dans l'autre, on veille à créer une surface aussi régulière que possible, et il est souhaitable d'avoir un faisceau aussi homogène que possible.

On retrouve ce souci lorsqu'il s'agit de modifier la courbure d'un disque réséqué prélevé sur l'oeil, par exemple selon la technique de Barraquer, ou d'usiner une lentille optique destinée à être accolée à la cornée de l'oeil à corriger ou insérée dans celle-ci.

On utilise généralement un laser pulsé à excimère, émettant à 193 nm, dont l'effet sur la cornée a été étudié pour la première fois par TABOADA J. et ARCHIBALD CJ: "An extreme sensitivity in the corneal epithelium to far UV ArF excimer laser pulses. Proceedings of the Scientific Program of the Aerospace Medical Association, San Antonio, Texas, 1981". L'utilisation d'un laser émettant dans le domaine des ultraviolets de courte longueur d'onde pour enlever du tissu humain a été décrite par R. SRINIVASAN (EP-A-0 111 060 IBM), et l'utilisation d'un tel laser en chirurgie cornéenne réfractive a été proposée par TROKEL SL et R. SRINIVASAN: "Excimer laser surgery of the cornea. American Journal of Ophtalmology December 1983; vol. 710-715".

Du fait que les ultraviolets de faible longueur d'onde ne peuvent pas parcourir les fibres optiques existantes sur une distance suffisante, on utilise des éléments optiques et un masque pour acheminer le faisceau laser à la surface de la cornée ou à la surface à modifier en fonction du profil d'ablation désiré, qui dépend du défaut de réfraction à corriger ou, en chirurgie cornéenne thérapeutique, de la profondeur des opacités cornéennes à ôter sans correction. Le profil d'ablation généralement considéré comme le plus approprié lorsqu'il s'agit de corriger un défaut de réfraction est parabolique et lisse. Toutefois, un profil sphérique est acceptable pour une faible correction et de petites zones d'ablation.

De nombreux types de dispositif d'acheminement du faisceau laser vers une cornée à traiter, généralement désigné par l'expression anglo-saxone "delivery system", basés sur un masque ont été conçus.

Pour la plupart, ils relèvent du principe de l'ouverture circulaire, selon lequel le profil d'ablation est obtenu soit par translation d'une ouverture circulaire de diamètre constant selon l'axe d'un faisceau focalisé (BIOPHYSIC MEDICAL WOA-86/04500), soit par la modification de diamètre d'une ouverture circulaire stationnaire (diaphragme constricteur décrit par MAINSTER MA : "Finding your way in the photo forest : laser effects for clinicians ; Ophtalmology 1984 ; 91: 886-888" et par MC DONALD et al : "Refractive surgery with excimer laser ; American Journal of Ophtalmology; 1987 ; 3:207"), soit par la rotation d'un disque à orifices (l'ESPERANCE EP-A-0 207 648), présentant successivement dans l'axe du faisceau des orifices circulaires de différentes tailles.

On a également proposé d'utiliser un masque à fente (HANNA-IBM EP-A-0 296 982), la forme de la fente étant liée au profil d'ablation. La correction est obtenue par balayage rotatif ou par un balayage combinant une rotation et une translation de l'image de la fente sur la surface de la cornée ou sur la surface à modifier ou à usiner.

Le type à ouverture circulaire présente l'inconvénient de donner un profil étagé à l'ablation, chaque impulsion ou succession d'impulsions avec un même diamètre d'ouverture donnant lieu à un gradin dont le diamètre est fonction respectivement de la position que l'ouverture mobile en translation occupe alors le long de l'axe du faisceau, du diamètre que présente alors l'ouverture stationnaire à diamètre variable ou du diamètre de l'orifice que le disque tournant présente alors dans le fais-ceau ; en outre, ce type ne peut s'appliquer qu'au traitement de la myopie, excepté en ce qui concerne le disque à orifices.

L'inconvénient du système à fente et balayage est qu'il requiert un grand degré de stabilité en ce qui concerne l'alignement du masque avec l'axe visuel.

Pour remédier à ces inconvénients, l'auteur de la présente invention a également proposé un masque-tamis, muni d'une multitude d'orifices mutuellement identiques, de diamètre comparativement petit, avec une densité proportionnelle au profil de l'ablation, une répartition aléatoire et un mouvement de rotation et/ou de translation du masque dans le faisceau (HANNA-IBM FR-A-2 655 837). Ce masque remédie effectivement aux inconvénients précités des masques précédemment connus, mais sa fabrication est délicate et coûteuse car elle impose l'utilisation d'un support en quartz opacifié localement par exemple par dépôt métallique. Cet inconvénient est d'autant plus sensible qu'un tel dépôt tend à disparaître lors de l'utilisation, si bien que la durée de vie de ce masque est réduite. En outre, il ne restitue qu'avec un certain degré d'incertitude, lors de son utilisation, le profil d'ablation déterminé en fonction duquel il a été conçu.

Le but de la présente invention est de proposer un masque qui, à la fois, offre une meilleure qualité d'ablation que le masque-tamis précité et puisse être fabriqué plus facilement et plus économiquement et bénéficier d'une durée de vie plus longue.

Ce but est atteint par le fait que le masque selon l'invention, destiné à être interposé dans un faisceau laser de longueur d'onde déterminée, avec possibilité de rotation autour d'un axe propre susceptible de coïncider avec un axe déterminé dudit faisceau, afin d'appliquer au moyen de celui-ci un traitement déterminé, notamment d'ablation superficielle selon un profil déterminé autour de l'axe du faisceau, à un organe ou un objet sécant de cet axe, notamment une cornée, une lentille ou un disque cornéen réséqué, le masque comportant à cet effet une zone opaque à ladite longueur d'onde et une zone transparente à celle-ci, présentant une forme et une position, par rapport audit axe propre, déterminées par une loi liant sa dimension angulaire, autour de celui-ci, à l'éloignement par rapport à celui-ci, de telle sorte qu'il en résulte la formation par le faisceau, sur ledit organe ou objet, d'une image de forme et de position, par rapport à l'axe du faisceau, déterminées en fonction dudit traitement, notamment dudit profil d'ablation, se caractérise en ce que la géométrie et la position, par rapport audit axe propre, de la zone transparente sont déterminées de telle sorte que celle-ci forme un ensemble fractal répondant à ladite loi.

La loi précitée est connue d'un Homme du métier ou aisément déterminable par un mathématicien-informaticien et ressort par exemple de EP-A-0 296 982 dans le cas d'une application à l'ablation cornéenne ou à l'usinage d'une lentille optique.

On sait que le concept d'ensemble fractal et la géométrie fractale a été développé par B. MANDELBROT ("Les objets fractals, Editions Flammarion, Paris, 1974-1984"; "The fractal geometry of nature, Editions W.H. Freeman and Company, San Francisco 1977-1982") qui, si l'on se limite à un espace à deux dimensions, caractérise notamment les fractals par la création, dans le contour d'une figure géométrique de type euclidien, d'irrégularités telles que le rapport de la longueur du contour de cette figure géométrique à sa surface tende vers l'infini.

Un Homme du métier comprendra aisément que lorsque la zone transparente d'un masque répond à cette définition, ce qui fait que l'image du faisceau sur la surface à traiter répond également à cette définition, les gradins qui résultent nécessairement, dans cette surface, de chaque impulsion du faisceau s'enchevêtrent progressivement en s'estompant mutuellement, au fur et à mesure de la succession des impulsions, accompagnée de la rotation du masque autour de l'axe du faisceau, et que l'on peut obtenir ainsi une surface d'ablation qui, par sommation d'irrégularités, présente finalement une grande régularité compte tenu des tolérances admissibles à cet égard.

Appliqué à la conception de masques pour "delivery system", conformément à la présente invention, ce concept peut présenter deux aspects alternatifs ou cumulatifs, si l'on suppose que l'on dessine la forme de la zone transparente à partir d'une forme euclidienne, régulière, correspondant par exemple à celle d'une fente répondant à la loi définie dans EP-A-0 296 982, ou à toute autre loi déterminée :
- on peut altérer cette forme régulière suivant une direction circonférentielle en référence à l'axe propre du masque, en préservant la dimension angulaire de cette forme en fonction de l'éloignement par rapport à cet axe,
- on peut également fractionner cette forme régulière suivant une direction circonférentielle, en référence à l'axe propre du masque, en préservant par cumul la dimension angulaire de cette forme en fonction de l'éloignement par rapport à l'axe propre du masque.

Dans un cas comme dans l'autre, la zone transparente obtenue ou les parties de zone transparente obtenues, considérées cumulativement, conservent à la fois la surface de la forme initiale de fente et la même répartition de cette surface dans le sens d'un éloignement par rapport à l'axe propre du masque, c'est-à-dire permettent d'obtenir le même profil d'ablation qu'avec la fente en question.

Un mathématicien-informaticien peut concevoir aisément d'autres méthodes pour dessiner la forme de la zone transparente à cet effet, notamment sans déterminer initialement une forme euclidienne répondant à la loi déterminée par le profil d'ablation souhaité pour traiter ensuite cette forme euclidienne ; toutefois, quelle que soit la méthode utilisée, la forme de la zone transparente obtenue correspond sensiblement, fût-ce par simple comparaison a posteriori, à une altération et/ou un fractionnement, suivant une direction circonférentielle en référence audit axe propre, d'une forme euclidienne répondant à ladite loi, avec préservation de la dimension angulaire de ladite forme euclidienne en référence audit axe propre, par cumul en cas de fractionnement, en fonction de l'éloignement par rapport audit axe propre, ce qui permet d'obtenir le même profil d'ablation qu'avec une fente qui présenterait ladite forme euclidienne.

En outre, en adoptant une forme fractale de zone transparente conformément à la présente invention, on peut donner à la zone transparente, respectivement à chaque partie de celle-ci en cas de fractionnement, des dimensions sensiblement supérieures à celles des orifices du masque-tamis décrit dans EP-A-0 296 982.

On peut ainsi choisir librement entre plusieurs procédés de fabrication, et notamment choisir une fabrication du masque sous la forme d'une plaque fine ajourée, par exemple métallique et obtenue par galvano-formation, moins coûteuse et plus durable qu'une lame transparente à la longueur d'onde du faisceau laser, et par exemple en quartz, opacifiée localement à cette longueur d'onde par un traitement, diélectrique ou autre, de surface, bien que l'on puisse également opter pour une telle constitution du masque.

Comme on le comprendra aisément à la lecture des ouvrages précités de B. MANDELBROT, la zone transparente peut présenter des formes très diverses, tout en répondant d'une part à la définition d'ensemble fractal et d'autre part à la condition d'illumination de l'organe ou de l'objet à traiter, avec rotation du masque sur lui-même, dans des conditions propres à procurer le profil d'ablation souhaité.

Selon une conformation actuellement préférée, la zone transparente peut ainsi présenter la forme de surfaces circulaires sécantes et/ou non sécantes, de diamètres variés.

Cependant, elle peut également présenter une forme ramifiée, ou encore la forme d'au moins une plage de contour irrégulier, mais ces exemples ne sont nullement limitatifs.

Naturellement, le mode de fabrication du masque est choisi de la façon la mieux appropriée en fonction de la forme choisie pour la zone transparente, étant entendu que dans le cas d'une fabrication sous forme d'une plaque fine ajourée, il est admissible de créer dans certaines parties de zone transparente, dont la forme ou l'étendue affaiblirait mécaniquement la plaque ou serait incompatible avec un tel mode de fabrication, des ponts de matière les traversant sans créer pour autant de perturbations sensibles dans la conformation du faisceau laser par le masque, de même que l'on peut renforcer ladite plaque par un doublage localisé dans la zone opaque, en retrait par rapport aux ajours matérialisant la zone transparente pour éviter une perturbation du faisceau qui les traverse.

Dans le cadre d'une application à la chirurgie réfractive, on peut ainsi réaliser des masques pour la correction de la myopie, caractérisés en ce que la dimension angulaire de la zone transparente, en référence à leur axe propre, est maximale à proximité de celui-ci, ou des masques pour la correction de l'hypermétropie, caractérisés en ce que la dimension angulaire de la zone transparente, en référence à leur axe propre, est maximale à proximité de la périphérie.

On peut également réaliser conformément à la présente invention des masques destinés à une ablation d'épaisseur ou profondeur constante ; un tel masque se caractérise en ce que la dimension angulaire de la zone transparente, en référence à l'axe propre du masque, est sensiblement constante de cet axe à la périphérie.

Naturellement, dans l'un et l'autre cas, on entend par "périphérie" la périphérie utile du masque, c'est-à-dire celle qui correspond au diamètre maximal que l'on puisse être amené à donner à l'image du faisceau laser sur la cornée, corrigé en fonction du grossissement du "delivery system" entre le masque et la cornée.

Dans tous les cas, la conformation de la zone transparente du masque pourra être aisément déterminée, par un mathématicien-informaticien, en fonction du profil d'ablation de surface souhaité.

Les masques selon l'invention, plus particulièrement conçus pour être utilisés en rotation autour de leur axe propre, notamment lorsqu'il coïncide avec l'axe du faisceau, sont de préférence également conçus pour réaliser un traitement identique dans toute section de l'organe ou de l'objet à traiter par un plan incluant cet axe, notamment pour réaliser des ablations selon un profil de révolution autour de cet axe ; toutefois, de façon particulièrement avantageuse, un masque ainsi conçu peut également être utilisé pour effectuer des traitements ne présentant pas une telle symétrie de révolution autour de l'axe du faisceau.

Ainsi, un masque prévu pour corriger la myopie ou l'hypermétropie sans astigmatisme peut être utilisé pour corriger en outre un astigmatisme.

A cet effet, la présente invention propose un procédé pour appliquer, au moyen d'un faisceau laser de longueur d'onde déterminée et d'axe déterminé, un traitement déterminé, notamment d'ablation superficielle selon un profil déterminé autour dudit axe, à un organe ou un objet sécant dudit axe, notamment une cornée ou une lentille destinée à être accolée à la cornée ou insérée dans celle-ci, ledit procédé consistant notamment à interposer un masque dans le faisceau et à le faire tourner autour de l'axe de celui-ci, par rapport à celui-ci, caractérisé en ce que l'on utilise un masque selon l'invention, présentant un plan moyen et un axe propre perpendiculaire à celui-ci, on oriente cet axe propre du masque par rapport à l'axe du faisceau de telle sorte qu'ils coïncident, notamment pour corriger une myopie ou une hypermétropie sans astigmatisme ou pour effectuer une ablation de profondeur constante, ou qu'ils forment entre eux un angle prédéterminé, autour d'un axe de pivotement relatif perpendiculaire à l'un et l'autre, notamment pour corriger un astigmatisme déterminé en faisant coïncider une image de l'axe de pivotement sur la cornée avec un axe d'astigmatisme, et on fait tourner le masque autour de l'axe du faisceau, par rapport à celui-ci, par rotation du masque autour de son axe propre par rapport au faisceau, pendant l'émission de celui-ci.

En d'autres termes, on effectue la correction de la myopie ou de l'hypermétropie sans astigmatisme, ou une ablation de profondeur constante, en orientant le masque perpendiculairement à l'axe du faisceau et en le faisant tourner autour de cet axe et on corrige la myopie ou l'hypermétropie avec astigmatisme en orientant le masque obliquement par rapport à une orientation perpendiculaire à l'axe du faisceau et en le faisant tourner autour de son axe propre.

De préférence, on choisit la disposition de la zone transparente, la vitesse et l'amplitude de rotation du masque et la fréquence des impulsions de telle sorte qu'en fin de traitement, c'est-à-dire lorsque cessent les impulsions, celles-ci aient correspondu à des positions de masque régulièrement réparties angulairement autour de l'axe du faisceau, d'une part, et que, dans toute la mesure du possible, des images successives identiques du faisceau ne se superposent jamais exactement sur l'organe ou l'objet à traiter, ce qui créerait des transitions trop nettes, difficiles à estomper par sommation des effets respectifs des impulsions successives, entre les zones traitées par illumination et les zones non traitées, à savoir des gradins trop prononcés.

A cet effet, on peut prévoir par exemple de faire tourner le masque d'un tour et dans un seul sens, autour de son axe propre, par rapport au faisceau, pendant l'émission de celui-ci, en synchronisant cette rotation avec les impulsions, dans le cas d'une émission par impulsions, afin que les impulsions soient émises alors que le masque occupe nécessairement des orientations différentes, régulièrement ou aléatoirement réparties angulairement autour de son axe propre.

Cependant, on peut également prévoir de faire tourner le masque en alternance dans un sens et dans le sens opposé, notamment de façon aléatoire, et/ou sur plus d'un tour autour de son axe propre, par rapport au faisceau, pendant l'émission de celui-ci.Dans un tel cas, on évite la coïncidence d'images successives identiques du faisceau sur l'organe ou objet à traiter en émettant le faisceau par impulsions et en synchronisant la rotation du masque autour de son axe propre, par rapport au faisceau, et lesdites impulsions de telle sorte que le masque occupe des positions angulaires différentes, autour de son axe propre, lors des différentes impulsions ; dans ce cas également, lorsqu'on les considère dans leur ensemble à la fin de l'émission des impulsions, ces positions sont régulièrement ou aléatoirement réparties angulairement autour de l'axe propre du masque.

En vue de la mise en oeuvre de ce procédé, la présente invention propose un dispositif spécifique.

Ce dispositif pour appliquer, au moyen d'un faisceau laser de longueur d'onde déterminée et d'axe déterminé, un traitement déterminé, notamment d'ablation superficielle selon un profil déterminé autour dudit axe, à un organe ou un objet sécant dudit axe, notamment une cornée ou une lentille destinée à être accolée à la cornée ou insérée dans celle-ci, ledit dispositif comportant notamment des moyens d'interposition d'un masque dans le faisceau avec possibilité de rotation autour de l'axe de celui-ci, par rapport à celui-ci, se caractérise en ce que, en vue de l'utilisation d'un masque selon l'invention, présentant un plan moyen et un axe propre perpendiculaire à celui-ci, les moyens d'interposition comportent :
- un bâti extérieur au faisceau, monté réglable en orientation par rapport au faisceau, autour de l'axe de celui-ci,
- un support extérieur au faisceau, monté réglable en orientation par rapport au bâti, autour d'un axe de pivotement perpendiculaire à l'axe du faisceau,
- une monture extérieure au faisceau, montée mobile en rotation sur le support, autour d'un axe de rotation perpendiculaire à l'axe de pivotement au même point que l'axe du faisceau, et susceptible de porter solidairement le masque dans une position dans laquelle l'axe de celui-ci coïncide avec l'axe de rotation,
- des moyens pour entraîner la monture en rotation par rapport au support, autour de l'axe de rotation, pendant l'émission du faisceau laser.

La rotation de la monture par rapport au support, autour de l'axe de rotation, peut s'effectuer sur un tour et dans un seul sens.

Cependant, on peut également prévoir que les moyens pour entraîner la monture en rotation soient propres à provoquer une rotation sur plus d'un tour et/ou en alternance dans des sens mutuellement opposés, notamment de façon aléatoire. Dans un tel cas, de préférence, le faisceau laser est émis par impulsions et les moyens pour entraîner la monture en rotation sont synchronisés avec les impulsions de telle sorte que la monture occupe des positions angulaires différentes par rapport au support, autour de l'axe de rotation, lors des différentes impulsions.

Dans un cas comme dans l'autre, lorsque le faisceau laser est émis par impulsions, les moyens pour entraîner la monture en rotation sont synchronisés avec les impulsions de telle sorte que celles-ci soient émises pour des orientations du masque différentes, régulièrement ou aléatoirement réparties angulairement autour de l'axe propre de celui-ci, selon une pratique connue en elle-même.

D'autres caractéristiques et avantages des différents aspects de l'invention ressortiront de la description ci-dessous, relative notamment à quelques exemples non limitatifs de masque, ainsi que des dessins annexés qui font partie intégrante de cette description ; en effet, la description complète des différentes formes de masque qui ont été illustrées nécessiterait des développements mathématiques importants alors que, en tout état de cause, ces développements mathématiques relèvent des aptitudes normales d'un mathématicien-informaticien dès lors que le profil d'ablation est donné, d'une part, et que de nombreuses autres formes de masques selon l'invention, aussi satisfaisantes, pourraient être conçues par ce mathématicien-informaticien, d'autre part.

Ces figures sont toutes relatives à une application des masques selon l'invention à la correction d'une amétropie par ablation superficielle localisée de la cornée, ou encore à l'ablation de la cornée sur une profondeur constante, par exemple pour éliminer des opacifications superficielles ou traiter une érosion récidivante, mais un Homme du métier comprendra aisément que ces masques peuvent trouver également leur application dans l'usinage de lentilles, d'origine naturelle, notamment prélevées sur une cornée, ou d'origine synthétique, par exemple en collagène, et destinées à être accolés à un oeil receveur ou insérées dans celui-ci, de même que l'on peut appliquer des masques selon l'invention à tout usinage d'objet par faisceau laser dans des conditions analogues à celles de telles interventions sur une cornée ou sur une lentille ; l'Homme du métier apportera aux dispositions qui vont être décrites les modifications nécessaires à chaque application, sans sortir pour autant du cadre de la présente invention.

Dans l'optique qui vient d'être définie :

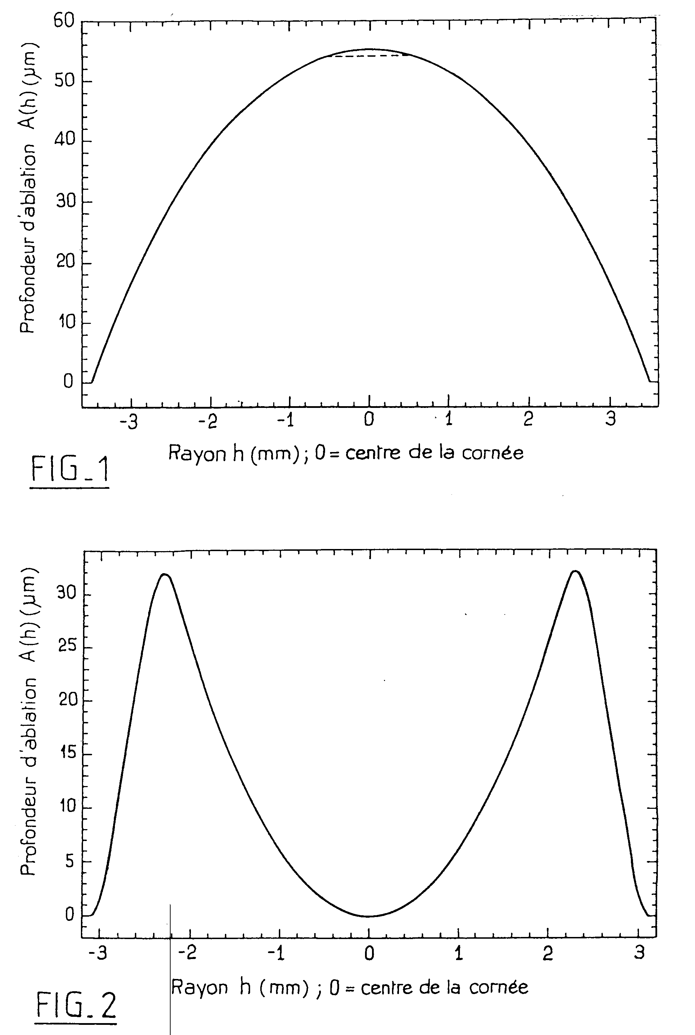

- les figures 1 et 2 montrent des profils d'ablation adaptés respectivement à la correction d'une myopie et à la correction d'une hypermétropie, en termes de profondeur d'ablation A (h) en micromètre, en fonction du rayon h, en millimètre, par rapport à l'axe visuel supposé passant par le centre de la cornée et confondu avec l'axe du faisceau laser ; naturellement, les chiffres ressortant de ces deux figures ne constituent que des exemples non limitatifs, notamment en ce qui concerne la profondeur maximale d'ablation, et doivent être adaptés au défaut de vergence à corriger dans chaque cas ;

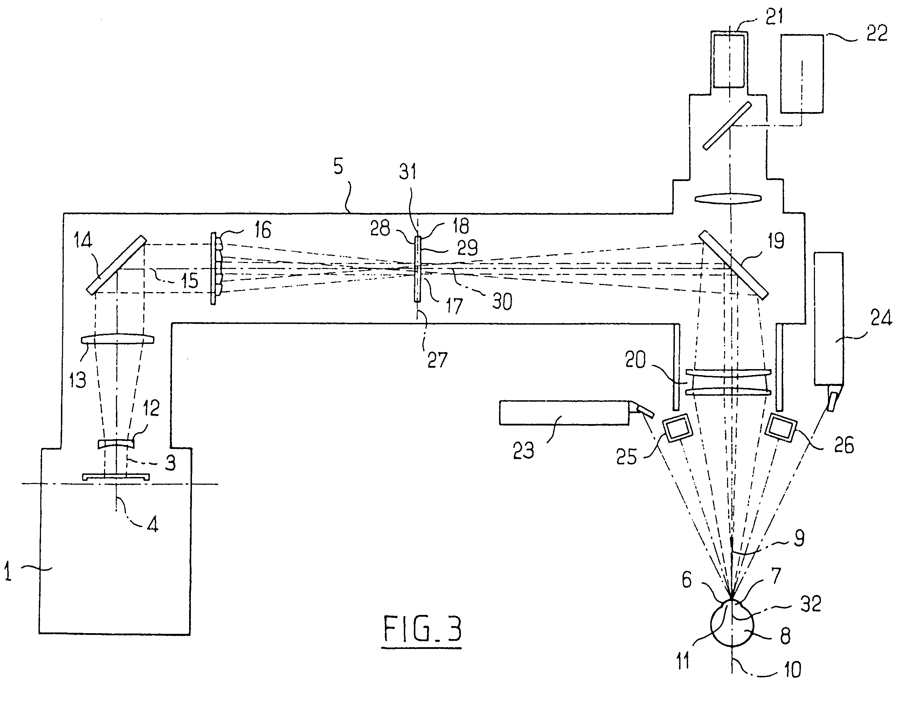

- la figure 3 illustre schématiquement un dispositif connu d'acheminement d'un faisceau laser vers une cornée à traiter, un tel dispositif étant généralement désigné par l'expression anglo-saxone "delivery-system" ;

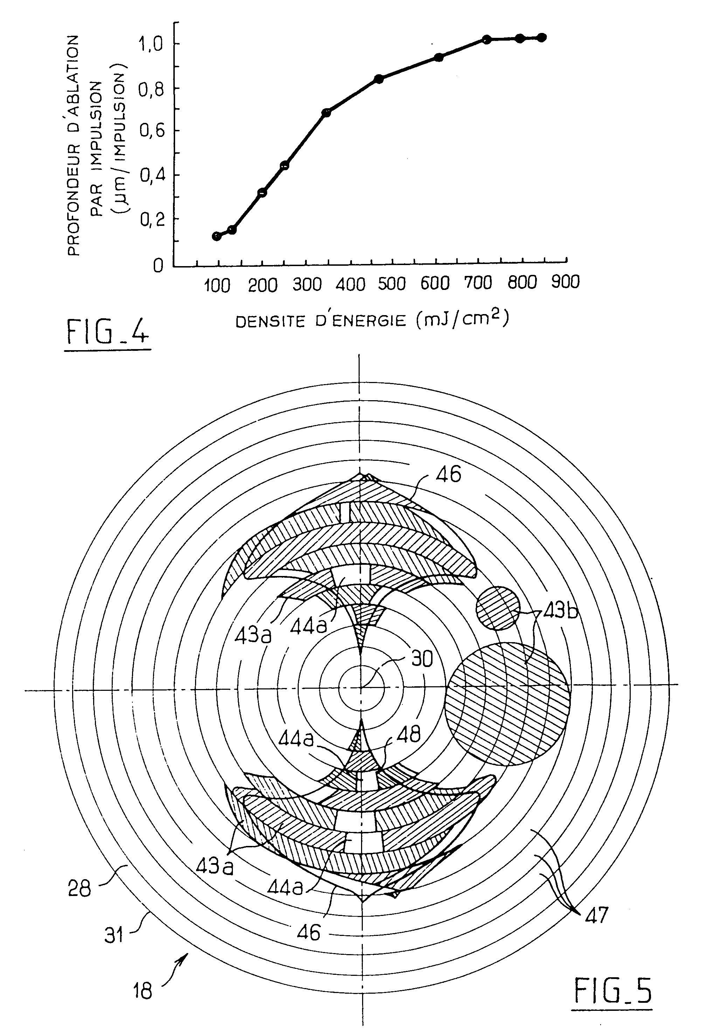

- la figure 4 illustre, également à titre de rappel, la profondeur d'ablation par impulsion, exprimée en micromètre par impulsion, en fonction de la densité d'énergie ou fluence, exprimée en millijoule par centimètre carré ;
- la figure 5 illustre schématiquement deux modes de transformation actuellement préférés d'une forme de fente, obtenue par exemple par application des enseignements de EP-A-0 296 982, en une forme fractale pour dessiner la zone transparente ou les parties de zone transparente d'un masque, conformément à la présente invention ;

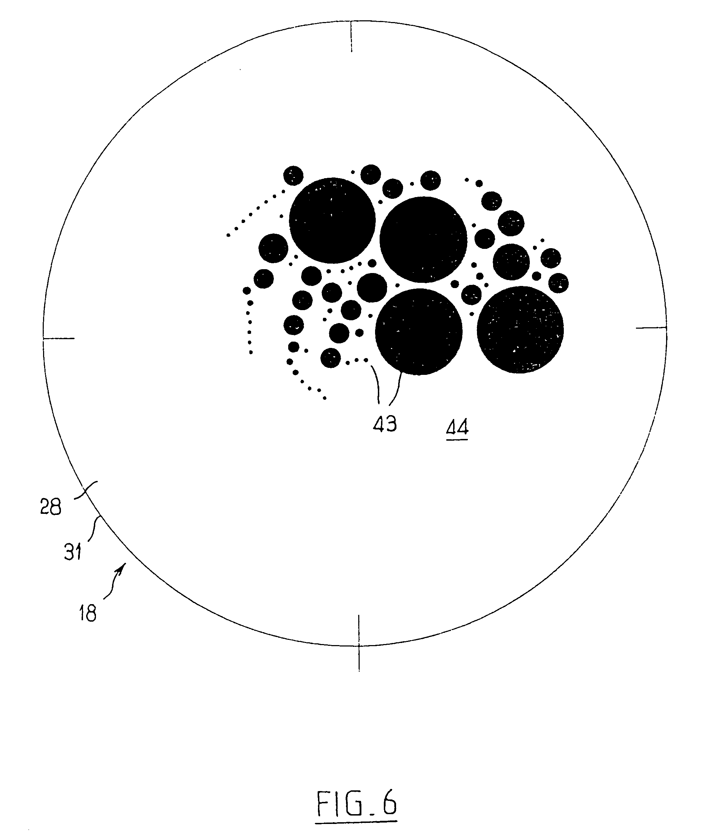

- les figures 6 à 14 illustrent, en des vues en plan, divers exemples de masques conformes à la présente invention et destinés à traiter une cornée pour corriger une myopie ou une hypermétropie, ou pour réaliser une ablation de profondeur ou épaisseur constante, en s'intercalant dans la trajectoire du faisceau laser à l'intérieur du "delivery system", dans une orientation perpendiculaire à un axe moyen du faisceau laser lorsqu'il n'y a pas lieu de corriger un astigmatisme ;

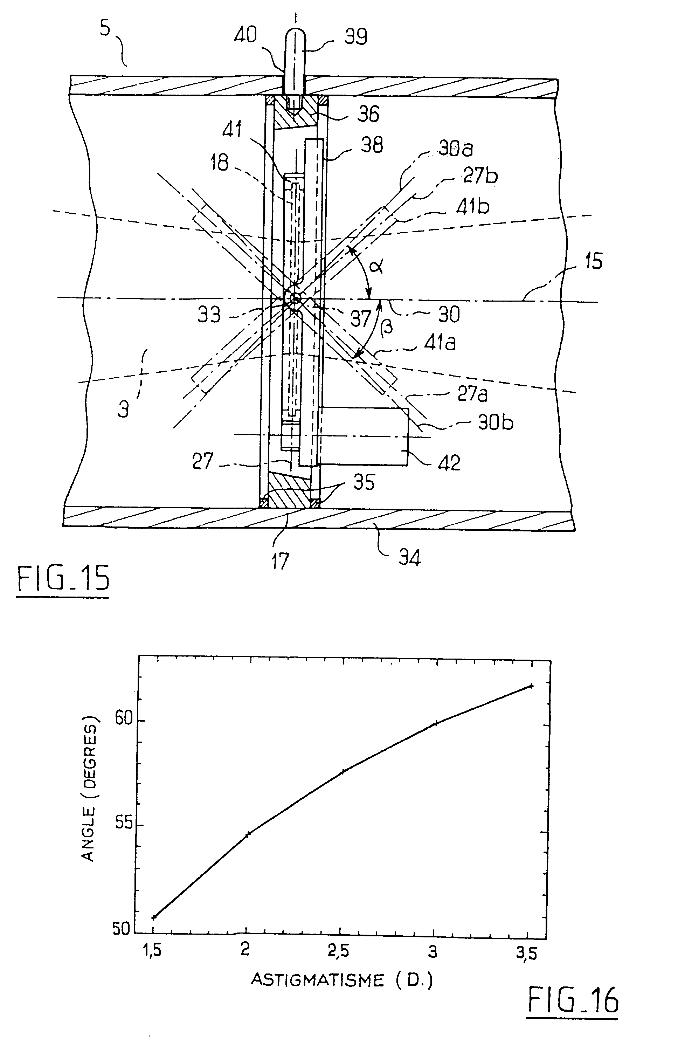

- la figure 15 illustre un montage de masque à l'intérieur du "delivery system", permettant d'utiliser également les masques illustrés aux figures 6 à 14 en leur donnant une orientation différente d'une orientation perpendiculaire par rapport à l'axe du faisceau laser, afin de corriger un astigmatisme, le cas échéant en complément de la correction d'une myopie ou d'une hypermétropie ;
- la figure 16 indique l'angle, en degrés, à communiquer à l'axe des masques par rapport à l'axe du faisceau pour corriger un astigmatisme donné, exprimé en dioptrie.

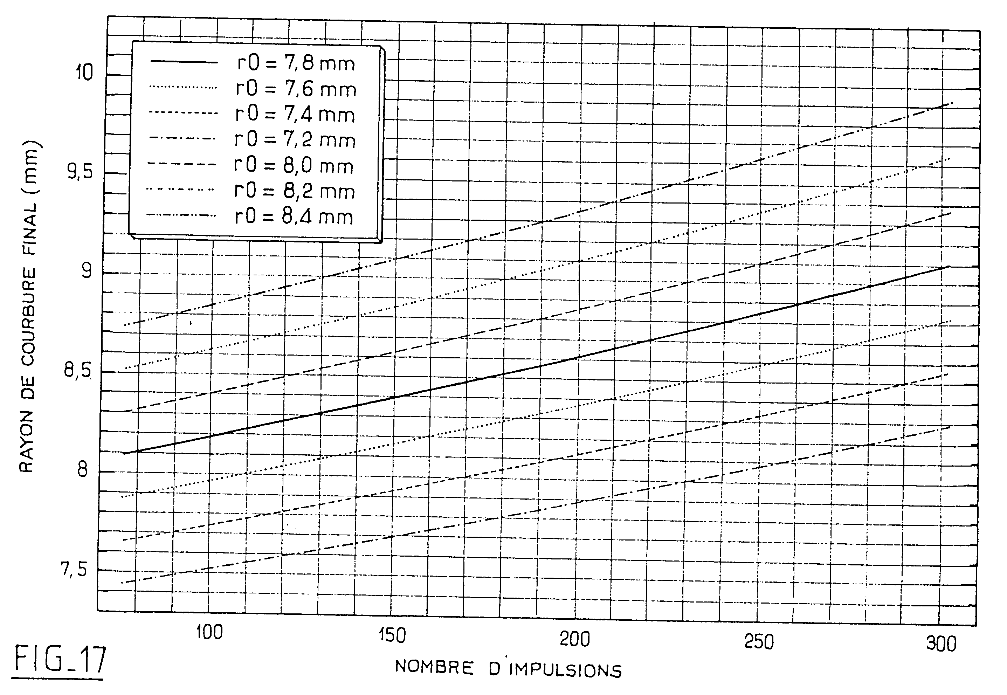

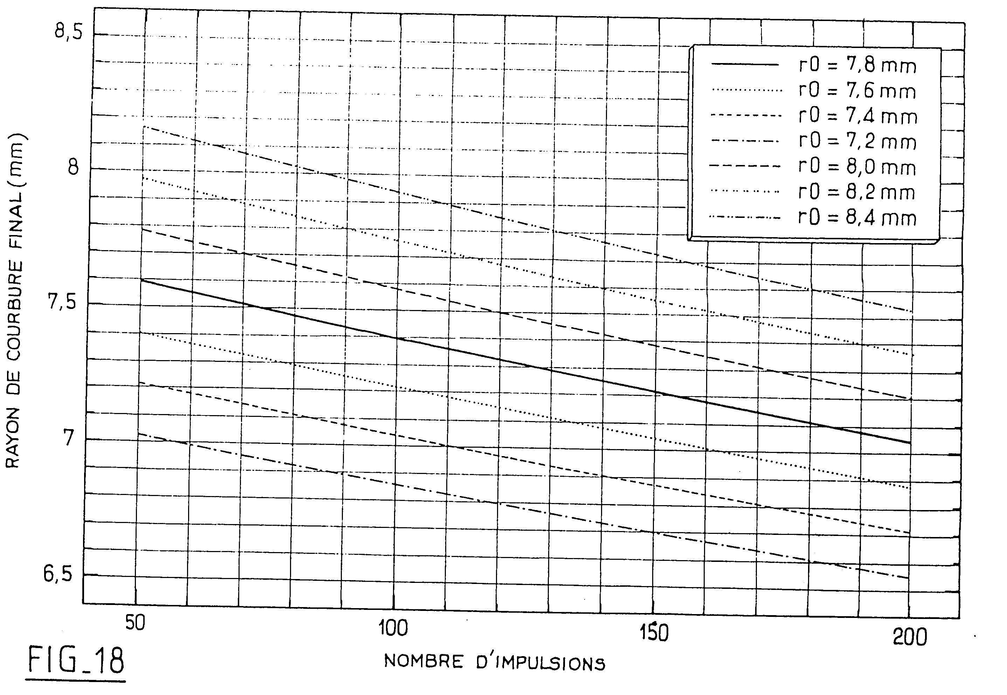

- les figures 17 et 18 sont des graphiques illustrant, respectivement dans le cas d'un masque déterminé destiné à la correction d'une myopie sans astigmatisme et dans le cas d'un masque déterminé destiné à la correction d'une hypermétropie sans astigmatisme, une relation existant entre le rayon de courbure initial de la face antérieure de la cornée sous forme d'une famille de droites, le nombre d'impulsions, en abscisse, et le rayon de courbure final de cette face antérieure, en ordonnées.

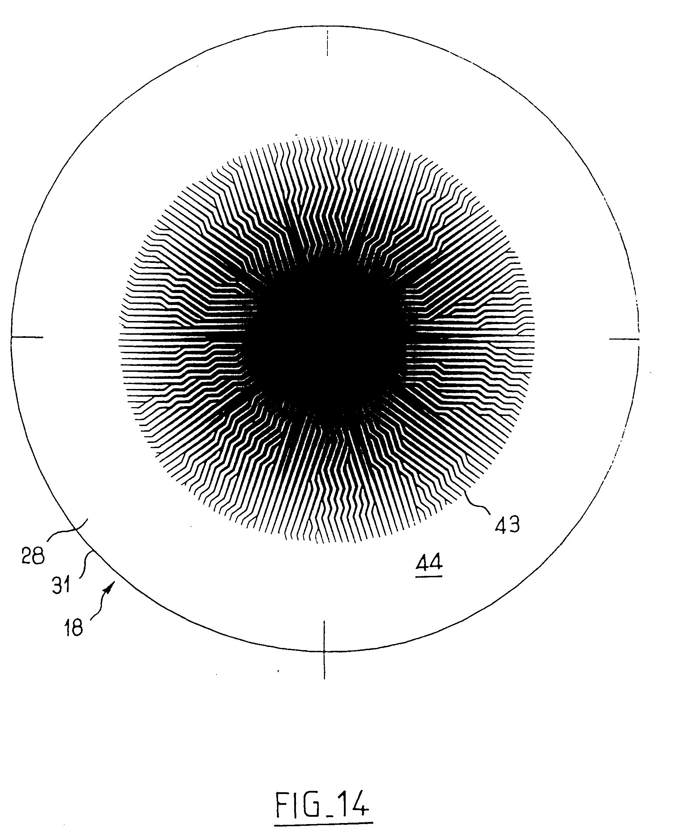

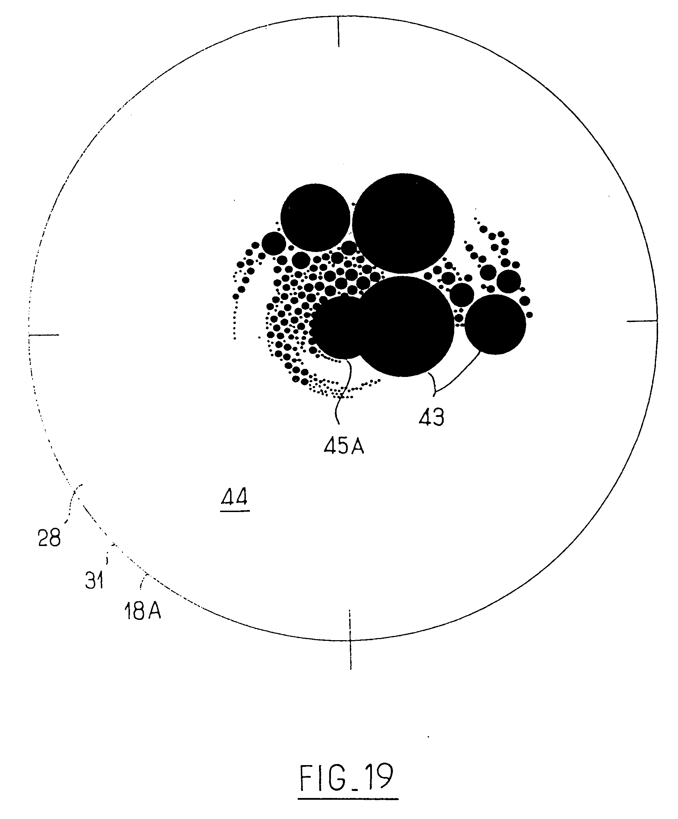

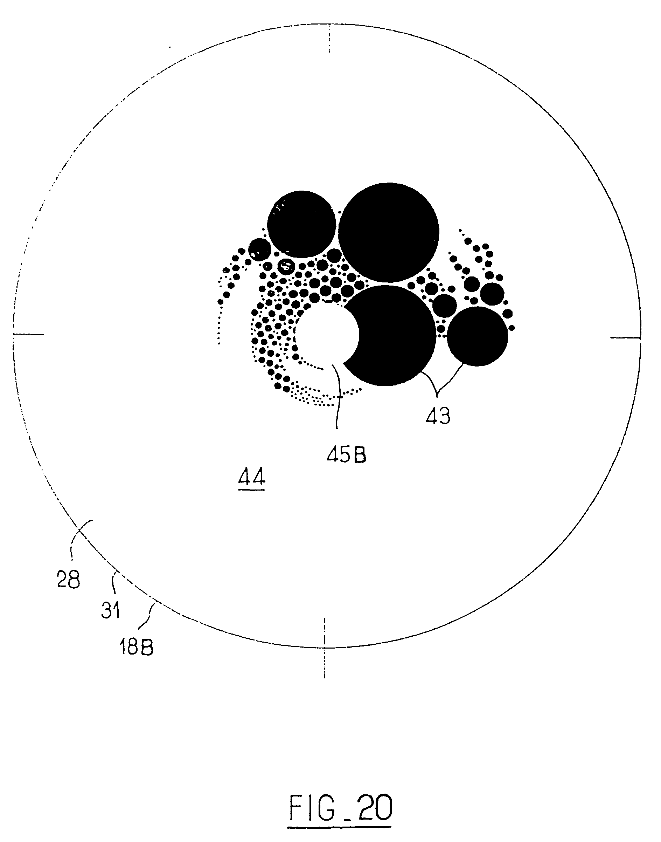

- les figures 19 et 20 illustrent, en une vue analogue à celle des figures 6 et 14, deux versions d'un exemple de masque conforme à la présente invention et destiné à traiter une cornée pour corriger une myopie, destinés à être utilisés à tour de rôle lors d'une intervention pour éviter une ablation aberrante au centre de la cornée lorsqu'un centrage mécanique précis du masque par rapport à son axe de rotation, à l'intérieur du "delivery system", est difficile à obtenir dans des conditions raisonnables de coût.

En se référant en premier lieu à la figure 1, on rappellera que lorsqu'il s'agit de corriger une myopie par ablation superficielle de la cornée, la profondeur ou épaisseur d'ablation est maximale au centre de la cornée, supposé coïncidant avec l'intersection de l'axe visuel avec la face antérieure de celle-ci, et décroît progressivement dans le sens d'un éloignement par rapport à ce centre ou à l'axe visuel, selon un profil sensiblement elliptique, jusqu'à s'annuler en dehors de la zone optique ; naturellement, la profondeur maximale d'ablation varie en fonction du défaut à corriger.

En se référant à présent à la figure 2, on rappellera que lorsqu'il s'agit de corriger une hypermétropie, la profondeur d'ablation minimale, éventuellement nulle, au centre de la cornée, croît progressivement dans le sens d'un éloignement par rapport à celui-ci ou à l'axe visuel, pour être maximale en dehors de la zone optique, de préférence selon un profil elliptique ; l'ablation a dans ce cas pour résultat l'aménagement d'une zone en retrait dans la face antérieure de la cornée, qui se raccorde à cette zone en retrait, en dehors de la zone optique, par une pente douce présentant une forme de révolution autour de l'axe visuel.

Dans le cas d'une correction de myopie comme dans le cas de la correction d'une hypermétropie, le profil d'ablation présente une forme de révolution autour de l'axe visuel lorsqu'il n'y a pas lieu de corriger un astigmatisme alors que, tout en conservant une forme elliptique dans tout plan de coupe incluant l'axe visuel, il présente des dimensions variant progressivement de l'un de ces plans de coupe à l'autre lorsqu'il s'agit de corriger un astigmatisme.

Dans la suite de la description, on se réfèrera à une correction de myopie ou d'hypermétropie sans astigmatisme, ou encore à une ablation de profondeur ou épaisseur constante, jusqu'à ce que les figures 15 et 16 spécifiquement orientées vers la correction d'un astigmatisme, notamment combiné avec une myopie ou une hypermétropie, soient commentées.

De façon connue, le laser à excimère émettant dans le domaine des ultraviolets d'une longueur d'onde de 193 nm constitue le moyen actuellement le mieux adapté, grâce à l'intercalation de masques appropriés sur la trajectoire du faisceau, pour réaliser un profil d'ablation elliptique du type illustré à la figure 1 ou du type illustré à la figure 2, ou encore du type, non illustré, correspondant à une profondeur d'ablation constante.

La figure 3 rappelle la conception générale du "delivery system" d'un tel laser.

Plus précisément, la figure 3 illustre, à titre d'exemple non limitatif de "delivery system" susceptible d'utiliser les masques selon l'invention, un système commercialisé par la Firme allemande Herbert Schwind GmbH & Co. à D8752 Kleinostheim, étant bien entendu que les masques selon l'invention pourraient être utilisés en combinaison avec tout autre type de "delivery system", et avec d'autres longueurs d'onde.

A la figure 3, on a désigné par 1 une source émettrice de rayonnement laser dans le domaine des ultraviolets d'une longueur d'onde de 193 nm, émettant un faisceau laser 3 suivant un axe 4, et l'on a désigné par 5 un "delivery system" acheminant ce faisceau 3 vers la face antérieure 6 de la cornée 7 d'un oeil 8 à traiter, suivant un axe 9 qui coïncide avec l'axe visuel 10 de cet oeil et coupe la face antérieure 6 de la cornée 7 sensiblement au centre 11 de cette face antérieure 6.

Dans l'exemple illustré, l'axe 9 est parallèle à l'axe 4 et le "delivery system" 5 comporte successivement, sur le trajet du faisceau laser 3, de la source 1 à la face antérieure 6 de la cornée 7 :
- une lentille divergente 12 qui rend divergent le faisceau 3, initialement parallèle, puis une lentille convergente 13 qui restitue le parallélisme de ce faisceau, qui conserve pour axe l'axe 4 à ce niveau,
- un miroir 14 de déviation à 90° qui, tout en conservant son parallélisme au faisceau, l'oriente suivant un axe 15 perpendiculaire à l'axe 4 ainsi qu'à l'axe 9,
- une lentille prismatique convergente 16 orientée perpendiculairement à l'axe 15 et concentrant le faisceau 3 en une zone 17 de l'axe 15,
- un masque selon l'invention 18, plat, orienté perpendiculairement au faisceau 15 dans la zone 17 lorsqu'il ne s'agit pas de corriger un astigmatisme, et de préférence monté en rotation autour de l'axe 15 à l'intérieur du "delivery system" 5, de façon motorisée au moyen d'un moteur qui sera décrit en référence à la figure 15, dans le cadre d'un montage autorisant la correction de l'astigmatisme,
- un miroir 19 transparent aux radiations visibles, déviant le faisceau 3 pour l'orienter suivant l'axe 9, vers la cornée 7 de l'oeil 8, en lui conservant la divergence qu'il présente après la zone 17,
- un condenseur optique 20, dont le faisceau 3 sort convergent vers la cornée 7 de l'oeil 8 à traiter, en sortant du "delivery system" 5.

A l'opposé du condenseur optique 20 par rapport au miroir 19, suivant l'axe 9, est disposé un ensemble de commande et de contrôle optique groupant un viseur 21 et une caméra vidéo 22 alors que, dans des positions mutuellement symétriques par rapport à l'axe 9 entre le condenseur optique 20 et la cornée 7 sont disposés, à la sortie du "delivery system" 5, d'une part deux lasers pilotes 23, 24 et d'autre part les deux objectifs 25, 26 d'un dispositif d'observation stéréoscopique orienté sensiblement vers le centre 11 de la face antérieure 6 de la cornée 7, dont la position par rapport au "delivery system" est prédéterminée, comme il est bien connu d'un Homme du métier.

Un tel Homme du métier comprendra également que le masque 18 selon l'invention se substitue aux masques traditionnellement utilisés dans l'Art antérieur, et pourra d'ailleurs équiper des "delivery system" 5 préexistants, quel que soit leur type, sans modification de ces derniers ou après des modifications simples et peu coûteuses.

Des exemples de réalisation de masque 18 selon l'invention vont être décrits à présent en référence à la figure 5 de façon générale et schématique quant au mode de dessin de leur zone transparente, et en référence aux figures 6 à 14, de façon spécifique, en relation avec leur application à un traitement d'ablation déterminé dans la face antérieure 6 de la cornée 7 de l'oeil 8, étant bien entendu que chacun de ces exemples devra être lui-même considéré comme représentatif d'une famille de masques 18, comportant une zone 43 transparente aux ultraviolets de la longueur d'onde utilisée, soit généralement de 193 nm, apparaissant en noir sur les dessins, répartie pour former un ensemble fractal sur le masque par ailleurs opaque aux ultraviolets de la longueur d'onde en question, soit 193 nm. En effet, il doit être bien entendu que la détermination des formes et emplacement de la zone transparente 43 ou des parties de zone transparente 43 par la méthode fractale peut conduire à un très grand nombre de solutions différentes, tout en offrant les mêmes possibilités d'application.

Quel que soit leur mode de réalisation, les masques 18 se présentent de préférence, comme il est illustré, sous une forme plate, délimitée par deux faces principales 28, 29 planes, mutuellement parallèles et mutuellement symétriques par rapport à un plan moyen 27 du masque 18, lequel plan moyen 27 est perpendiculaire à l'axe 15 lorsque, comme on l'a illustré à la figure 3, il s'agit de pratiquer une ablation sans correction d'astigmatisme. Les deux faces principales 28 et 29 présentent elles-mêmes un axe commun 30 qui leur est perpendiculaire et se confond alors avec l'axe 15 ; cet axe 30 constitue pour les masques 18 un axe moyen et les faces 28 et 29 sont par exemple raccordées mutuellement par un chant 31 cylindrique de révolution autour de cet axe 30 et définissant leur périphérie.

Selon les cas, la zone 43 transparente à la longueur d'onde en question et la zone 44 opaque à cette longueur d'onde peuvent être constituées respectivement par des ajours et par des pleins d'une plaque métallique, par exemple en nickel, obtenue par exemple par galvano-formation, ou constituer respectivement des zones non traitées et des zones convenablement traitées d'une lame d'un matériau transparent aux ultraviolets présentant la longueur d'onde en question, et par exemple d'une lame de quartz. Il est bien entendu toutefois que ces modes de réalisation, en particulier ces matériaux, ne constituent que des exemples non limitatifs et qu'un Homme du métier pourra choisir d'autres matériaux appropriés, de même que tout autre mode approprié de réalisation des masques 18, sans pour autant sortir du cadre de la présente invention.

Dans le cas d'une fabrication des masques sous forme d'une plaque métallique, on pourra être amené, pour éviter une trop grande vulnérabilité de celle-ci, à fixer cette plaque sur des moyens de renforcement présentant par exemple la forme d'une plaque plus épaisse et plus rigide, fixée à plat contre l'une de ses faces principales 28, 29 et présentant des découpes correspondant aux ajours définissant la zone transparente mais de dimensions supérieures afin que leurs bords n'interfèrent pas avec ceux des ajours. On peut également, à cet effet, emprisonner ladite plaque métallique entre deux plaques plus épaisses et plus rigides, présentant de telles découpes. On pourra également être amené, à cet effet, à maintenir des ponts de matière à travers les ajours correspondant à certaines parties de zone transparentes, pour rompre leur continuité et maintenir une cohérence à la plaque.

Les figures 6 à 8 montrent des masques 18 dont la zone transparente 43 présente la forme d'un groupe de surfaces circulaires de diamètres divers, sécantes ou non, appelées "trous" par référence à un mode de réalisation du masque sous forme d'une plaque fine ajourée et qui sont destinés respectivement à la correction de la myopie, la correction de l'hypermétropie, et l'ablation sur une épaisseur constante à partir de la face antérieure 6 de la cornée 7. La forme des parties de zone transparente 43 s'apparente dans ce cas à celle de bulles de savon, considérées dans un plan, ou à celle de trous dans un fromage, également considérés dans un plan.

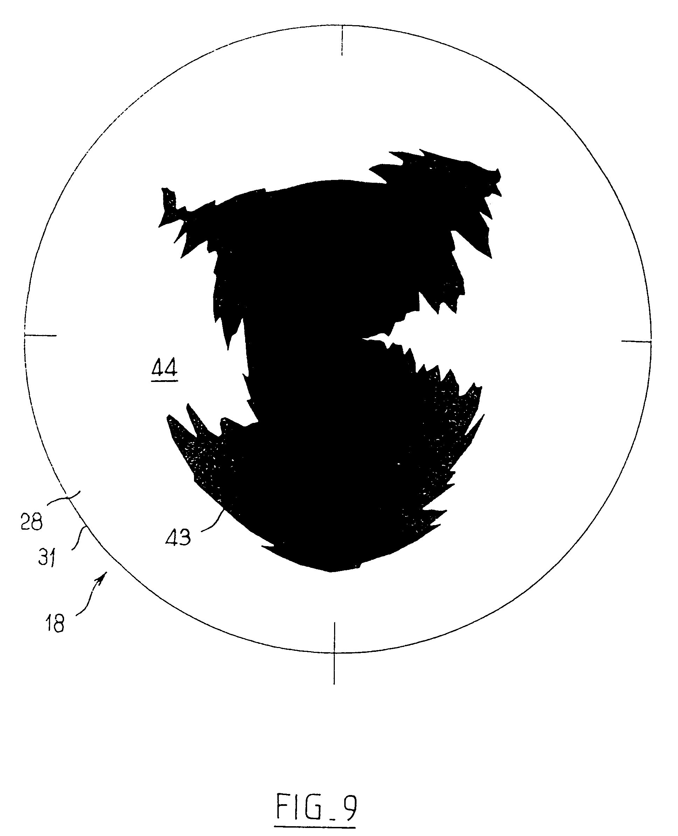

Les figures 9 à 11 correspondent au cas de masques 18 dont la zone transparente 43 présente la forme d'une plage ou d'un continent aux contours irréguliers et qui correspondent respectivement à la correction de la myopie, à la correction de l'hypermétropie et à l'ablation sur une épaisseur constante.

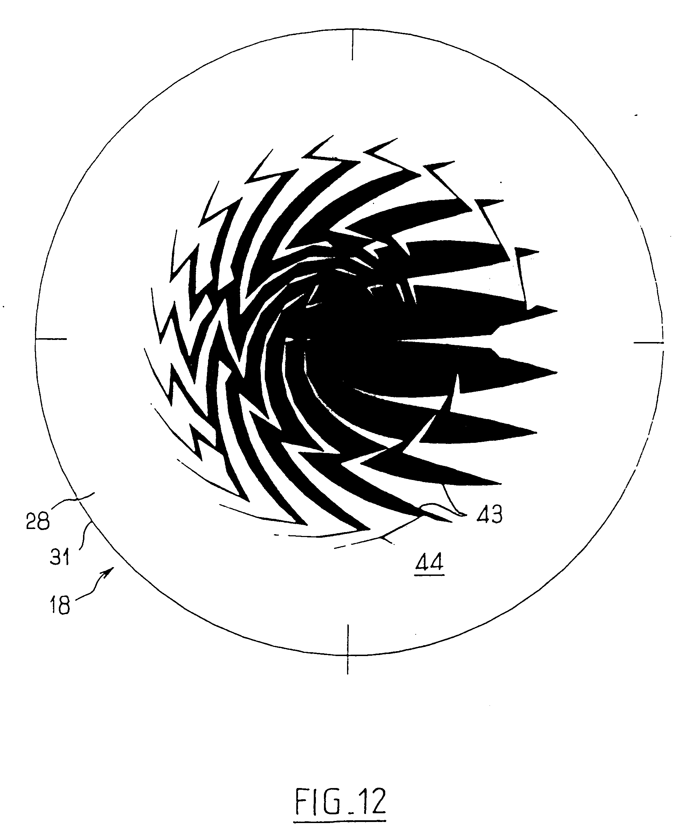

Les figures 12 à 14 illustrent des masques 18 dont la zone transparente 43 est ramifiée et qui sont destinés à la correction de la myopie. Géométriquement, il s'agit d'une variante du masque illustré à la figure 9, comme le comprendra aisément un spécialiste de la géométrie fractale, qui comprendra également que l'on pourrait réaliser également sous forme ramifiée des masques destinés à la correction de l'hypermétropie et des masques destinés à une ablation sur une épaisseur constante.

La forme de la zone transparente 43 de chacun de ces masques 18 est générée au moyen d'un programme informatique, selon l'une ou l'autre de deux méthodes qui vont être décrites en premier lieu en référence à la figure 5, où l'on retrouve les références numériques 18, 28, 30, 31, pour désigner respectivement un masque, l'une de ses faces principales, par exemple celle qui est tournée vers la lentille 16 dans le "delivery system" 5, l'axe propre du masque 18 et le chant de celui-ci, lequel chant sera considéré comme délimitant la zone utile susceptible d'être traversée par le faisceau 3 ou d'intercepter celui-ci ; on a par ailleurs désigné respectivement par 43a et 43b des parties de zone transparente obtenues respectivement par l'une et l'autre des méthodes qui vont être décrites à présent, et par 44 la zone opaque, étant entendu que ces notions de transparence et d'opacité s'entendent par référence à la longueur d'onde du faisceau laser utilisé.

Une étape initiale commune aux deux méthodes consiste à déterminer le profil d'ablation à obtenir, conformément à la figure 1 ou à la figure 2 selon qu'il s'agit de corriger une myopie ou une hypermétropie ou encore de façon non illustrée puisque se présentant sous forme d'une simple droite parallèle à l'axe des abscisses lorsqu'il s'agit d'effectuer une ablation de profondeur constante, puis à dessiner par une méthode traditionnelle, par exemple la méthode décrite dans EP-A-0 296 982, la forme d'une fente unique ou de chacune des fentes d'une famille de fentes qui permettrait d'obtenir ce profil d'ablation avec un masque rotatif à fente de l'Art antérieur, du type décrit dans EP-A-0 296 982.

On a illustré à la figure 5, sans chercher à se référer à un profil d'ablation particulier, une forme de fente 46 ainsi obtenue, prévue en deux exemplaires mutuellement symétriques par rapport à l'axe 30 du masque.

Il est bien entendu, cependant, que la méthode traditionnelle appliquée pourrait aboutir à prévoir une seule fente, ou plus de deux fentes, et les formes de fente différentes de la forme illustrée.

Chaque forme de fente ainsi dessinée présente une répartition déterminée de sa surface dans le sens d'un éloignement par rapport à l'axe 30, ce que l'on peut exprimer par le fait qu'elle présente une dimension angulaire déterminée, par rapport à cet axe, en fonction de l'éloignement par rapport à celui-ci.

A partir de la ou chaque forme de fente ainsi dessinée, ou encore à partir des formes de fente ainsi dessinées et considérées dans leur globalité, on peut dessiner les zones transparentes d'un masque selon l'invention par la succession des étapes suivantes, schématisées à la figure 5, de préférence de façon informatisée.

Une première étape consiste à subdiviser géométriquement la surface utile du masque, illustrée par la face 28 de celui-ci à la figure 5, sous forme d'anneaux concentriques 47 centrés sur l'axe 30 et répartis de façon uniforme ou non de celui-ci à la périphérie 31 du masque. Les anneaux 47 fractionnent chaque forme de fente en plusieurs parties de zone transparente 43a et il est bien entendu que le fractionnement de la ou chaque forme de fente 46 sera d'autant plus grand que le nombre d'anneaux 47 sera grand.

Ensuite, en considérant successivement chaque partie de forme de fente 46 délimitée par un anneau 47, on décale angulairement cette partie autour de l'axe 30, par exemple de façon pseudo-aléatoire, sans autre déplacement, ce qui préserve à la fois sa dimension angulaire en référence à l'axe 30 et son éloignement par rapport à celui-ci. Ce déplacement de chaque partie peut s'effectuer sans division de celle-ci, ou encore après une subdivision en plusieurs sous-parties que l'on décale angulairement de façon différente autour de l'axe 30, étant entendu que leur dimension angulaire cumulée autour de cet axe reste identique à celui de la zone de la forme de fente 46 dont elles sont issues. Il est bien entendu que certaines parties de la forme de fente 46, correspondant à certains anneaux 47, peuvent également ne pas être déplacées.

Lorsque plusieurs formes de fente 46 sont prévues, comme c'est le cas dans l'exemple illustré à la figure 5, ces formes de fente 46 peuvent être traitées indépendamment l'une de l'autre, ce qui signifie que leurs parties correspondant à un même anneau peuvent subir des décalages angulaires différents autour de l'axe 30, de même que l'on peut regrouper les parties de l'une et l'autre correspondant à un même anneau, le cas échéant en les divisant.

Un Homme du métier comprendra aisément, au simple examen de la figure 5, combien la subdivision de la forme de fente 46 en parties 43a de zone transparente, selon ce premier mode de fractionnement, permet d'augmenter le contour de la ou chaque zone transparente tout en préservant la même surface cumulée de zone transparente et la même répartition de cette surface cumulée dans le sens d'un éloignement par rapport à l'axe 30.

Dans les commentaires, ci-dessous, relatifs aux masques illustrés aux figures 9 à 18, dont les zones transparentes 43 sont obtenues par cette méthode, on a appelé "zone de fractionnement" chaque forme de fente 46, calculée par exemple par la méthode indiquée dans EP-A-0 296 982, et "bifurcation" chaque fractionnement angulaire, autour de l'axe 30, d'une zone de forme de fente 46 située à l'intérieur d'un anneau 47.

On observera qu'un tel fractionnement peut laisser apparaître entre certaines parties de zone transparente 43a des parties 44a de zone opaque qui ne sont pas reliées aux autres parties de zone opaque. Celles-ci ne présentent aucune difficulté de fabrication lorsque le masque est réalisé par traitement de surface d'une plaque d'un matériau perméable aux ultraviolets de la longueur d'onde utilisée, mais aboutit à une impossibilité de fabrication dans le cas de masques réalisés sous forme d'une plaque obtenue par exemple par galvano-formation ; dans un tel cas, on peut prévoir que les parties de zone opaque 44a soient reliées mutuellement par de fins ponts de matière 48 traversant les parties de zone transparente 43a sans créer de perturbation sensible dans l'effet obtenu au moyen du masque.

La figure 5 illustre un autre mode de fractionnement de chaque forme de fente 46 pour réaliser un masque selon l'invention, et les figures 6 à 8 illustrent des masques obtenus par cette autre méthode.

Comme la précédente, cette méthode peut être avantageusement informatisée.

Cette autre méthode consiste à subdiviser chaque forme de fente 46, ou les formes de fente 46 considérées dans leur ensemble, c'est-à-dire quant à la répartition de leur surface cumulée dans le sens d'un éloignement autour de l'axe 30 ou encore quant à leur dimension angulaire cumulée autour de cet axe 30, en fonction de l'éloignement par rapport à celui-ci, en parties transparentes 43b en forme de disque circulaire.

A cet effet, une méthode utilisée avec satisfaction consiste à déterminer le diamètre et la position, dans le sens d'un éloignement par rapport à l'axe 30, d'une partie 43b de surface transparente de contour circulaire qui permettrait d'obtenir, de façon convenablement positionnée sur la face antérieure 7 de la cornée 6, une ablation correspondant à l'ablation maximale à effectuer, ressortant du profil d'ablation illustré à la figure 1 ou à la figure 2 selon qu'il y aurait lieu de corriger une myopie ou une hypermétropie. Ensuite, mathématiquement, on détermine quel devrait être le profil d'ablation résiduaire à adopter si, en partant du profil d'ablation à réaliser tel qu'il est illustré à la figure 1 ou 2, on avait déjà effectué une ablation en utilisant un masque comportant, à titre de fente, la partie 43b de zone transparente ainsi définie, puis on détermine de la même manière la forme et la position, dans le sens d'un éloignement par rapport à l'axe 30, d'une partie de zone transparente 43b qui permettrait, si l'on utilisait un masque la présentant à titre de zone transparente, d'effectuer l'ablation maximale ressortant de ce profil d'ablation résiduaire, et l'on répète l'opération jusqu'à ce que, avec une approximation suffisante, le profil d'ablation obtenu grâce à l'ensemble des parties 43b de zone transparente corresponde au profil d'ablation souhaité.

Naturellement, cette méthode conserve une répartition identique des surfaces cumulées ou dimensions angulaires cumulées, en référence à l'axe 30, des parties 43b de zone transparente en fonction de l'éloignement par rapport à l'axe 30.

Une autre méthode, aboutissant au même résultat, consiste à dessiner une première partie 43b de zone transparente de contour circulaire qui corresponde au plus grand cercle susceptible d'être inscrit à l'intérieur de chaque forme de fente 46, puis des parties circulaires 43b de zone transparente de plus en plus petites pour couvrir au maximum la surface de la forme de fente 46 en réalisant à l'intérieur de cette forme ce que l'on appelle un bourrage apollonien de cercles, avant de répartir ces cercles angulairement autour de l'axe 30 en conservant à chacun d'eux son éloignement par rapport à celui-ci. On peut également traiter ainsi une forme de fente unique, regroupant les différentes formes de fente dessinées.

Dans l'un et l'autre cas, on se fixe naturellement un diamètre minimal des cercles, c'est-à-dire des trous après réalisation du masque s'il est réalisé sous forme d'une plaque ajourée, leur diamètre maximal ainsi qu'un écartement mutuel minimal entre eux.

Dans les commentaires ci-dessous, relatifs spécifiquement à chacun des masques illustrés aux figures 6 à 14 et à un mode de réalisation par électro-formation, on a fait apparaître successivement :
- le diamètre de la zone d'ablation sur la cornée 7 à corriger, mesuré autour du centre 11 de cette cornée ou de l'axe visuel 10, étant entendu que l'on utilisera des masques 18 différents en fonction du diamètre de cette zone optique ;
- le diamètre du masque 18, c'est-à-dire son diamètre utile à l'intérieur des moyens, non illustrés à la figure 3, mais détaillés ultérieurement en référence à la figure 15, qui assurent son montage à l'intérieur du "delivery system" 5 ; les figures 6 à 14 correspondant à des cas dans lesquels ce diamètre est celui du chant 31 du masque ;
- le nombre de trous avec leur diamètre minimal et leur diamètre maximal dans le cas des figures 6 à 8 ou le nombre d'anneaux, de zones de fractionnement et de bifurcations, avec le diamètre d'une zone centrale éventuellement occultée du masque, mesuré comme le diamètre précité de celui-ci en référence à son axe 30, dans le cas des figures 9 à 14 ;
- la surface occupée par les trous ;
- le rayon de courbure initial de la cornée à corriger, mesuré au niveau de sa face antérieure 6 en référence à un centre 32 situé à l'intérieur de l'oeil 8, au moins approximativement sur l'axe visuel 10 ;
- le rayon de courbure final de cette cornée, mesuré dans des conditions analogues, après un nombre déterminé d'impulsions laser accompagnées d'une rotation du masque autour de son axe 30 alors confondu avec l'axe 15 ; et
- l'épaisseur d'ablation maximale mesurée en référence à la face antérieure 6 de la cornée, suivant l'axe visuel 10 ou parallèlement à celui-ci.

La notion de trous serait à remplacer par la notion plus générale de zones transparentes à la longueur d'onde utilisée dans ce cas d'autres modes de réalisation, notamment dans le cas d'une réalisation sous forme d'une lame transparente localement opacifiée.
**EXEMPLE 1** (figure 6) : correction de la myopie :

| | |
|---|---|
| diamètre de la zone d'ablation : | 7,00 mm |
| diamètre du masque : | 10,45 mm |
| nombre de trous : | 79 |
| ⌀ 100,0 µm minimum | 2100,0 µm maximum |
| | écartement mutuel : 120,0 µm |
| surface occupée par les trous : | 22,98% |
| rayon de courbure initial = | 7,80 mm |
| rayon de courbure final = | 8,30 mm après 500 impulsions |
| épaisseur d'ablation maximale = | 55,30 µm tolérance : 1,00% |

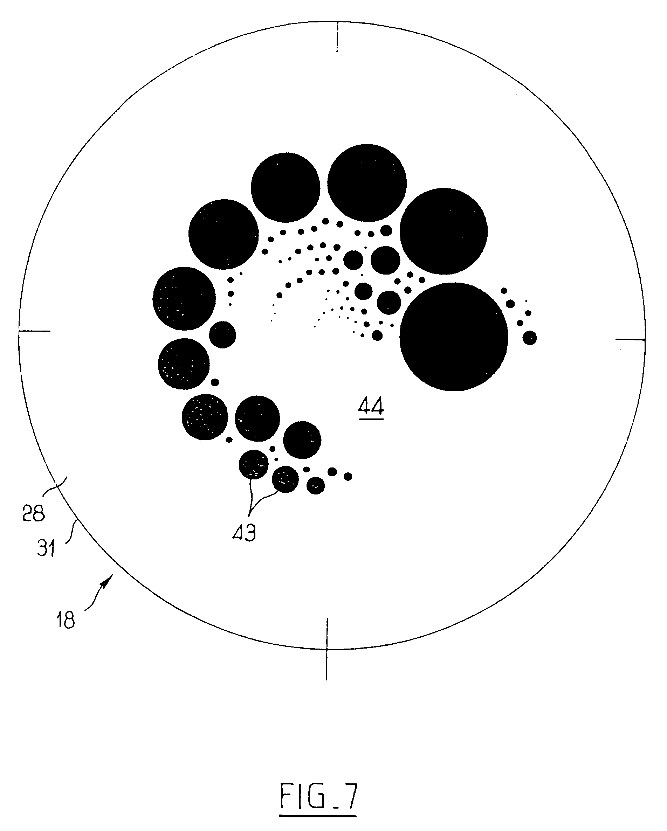

**EXEMPLE 2** (figure 7) : correction de l'hypermétropie :

| | |
|---|---|
| diamètre de la zone d'ablation : | 6,50 mm |
| diamètre du masque : | 9,70 mm |
| nombre de trous | 89 |
| ⌀ 31,1 µm minimum | 2600,0 µm maximum |
| | écartement mutuel : 150,0 µm |
| surface occupée par les trous : | 34,12% |
| rayon de courbure initial = | 8,10 mm |
| rayon de courbure final = | 7,80 mm après 80 impulsions |
| épaisseur d'ablation maximale = | 13,70 µm tolérance : 1,50% |

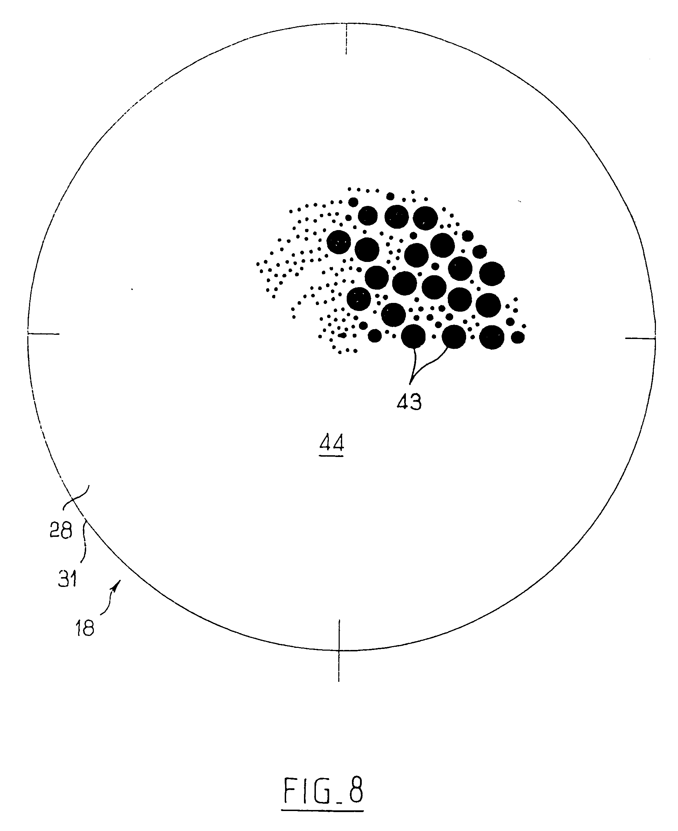

**EXEMPLE 3** (figure 8) : masque neutre :

| | |
|---|---|
| diamètre de la zone d'ablation : | 6,00 mm |
| diamètre du masque : | 8,96 mm |
| nombre de trous | 181 |
| ⌀ 100,0 µm minimum | 600,0 µm maximum |
| surface occupée par les trous : | 11,33% |
| rayon de courbure initial = | 8,00 mm |
| rayon de courbure final = | 8,00 mm après 5000 impulsions |
| épaisseur d'ablation maximale = | 200,02 µm |

**EXEMPLE 4** (figure 9) : correction de la myopie :

| | |
|---|---|
| diamètre de la zone d'ablation : | 7,50 mm |
| diamètre du masque : | 11,19 mm |
| nombre d'anneaux : | 50 sur 1 zone 2 bifurcations |
| Diamètre zone centrale : | 0,50 mm |
| surface occupée par les trous : | 52,02% |
| rayon de courbure initial = | 7,80 mm |
| rayon de courbure final = | 8,30 mm après 260 impulsions |
| épaisseur d'ablation maximale = | 64,91 µm |

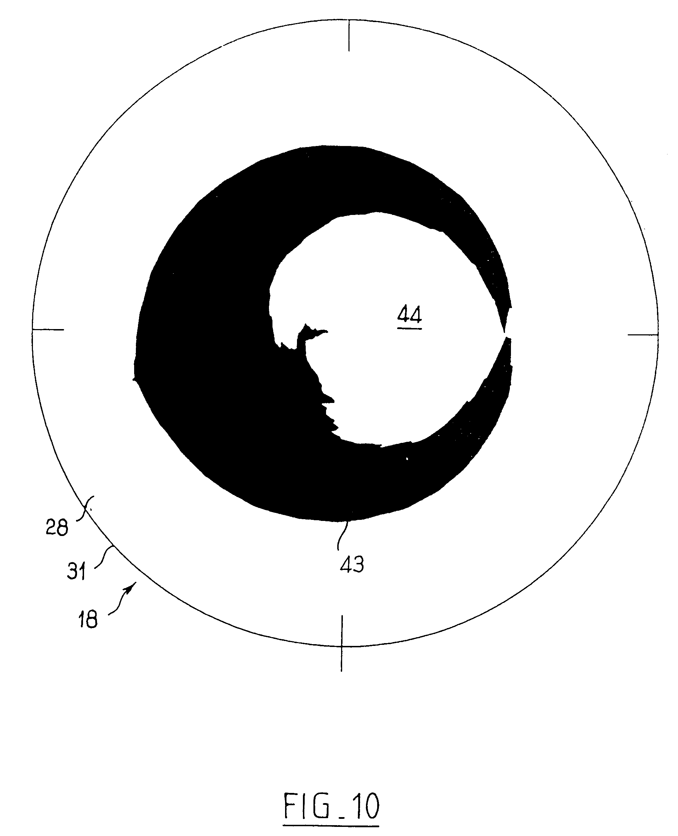

**EXEMPLE 5** (figure 10) : correction de l'hypermétropie :

| | |
|---|---|
| diamètre de la zone d'ablation : | 7,00 mm |
| diamètre du masque : | 10,45 mm |
| nombre d'anneaux : | 100 sur 1 zone 0 bifurcation |
| diamètre zone centrale : | 0,01 mm |
| surface occupée par les trous : | 48,84% |
| rayon de courbure initial = | 8,30 mm |
| rayon de courbure final = | 7,80 mm après 104 impulsions |
| épaisseur d'ablation maximale = | 26,09 µm |

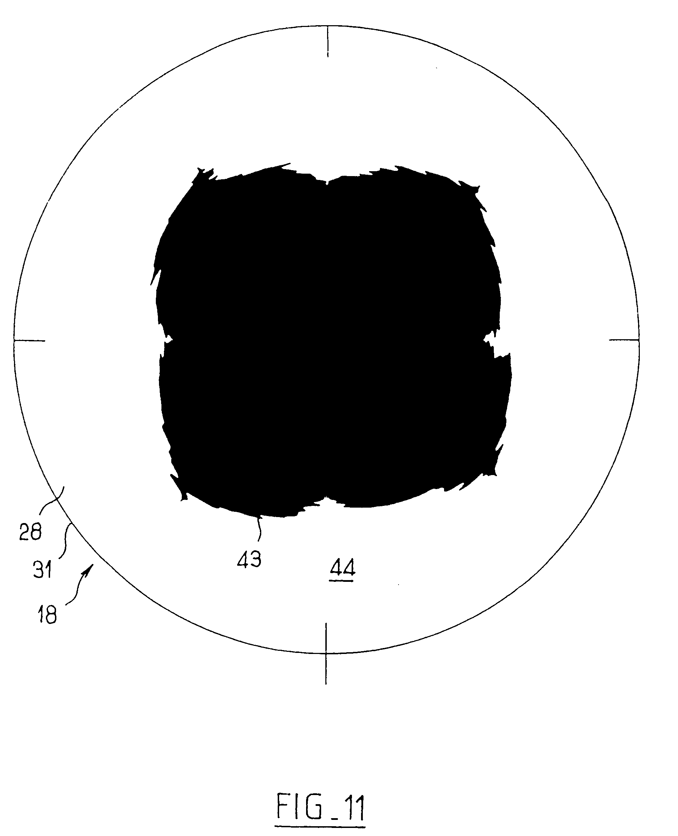

**EXEMPLE 6** (figure 11) : masque neutre :

| | |
|---|---|
| diamètre de la zone d'ablation : | 7,00 mm |
| diamètre du masque : | 10,45 mm |
| nombre d'anneaux : | 100 sur 4 zones 0 bifurcation |
| diamètre zone centrale : | 0,01 mm |
| surface occupée par les trous : | 72,04% |
| rayon de courbure initial = | 7,80 mm |
| rayon de courbure final = | 7,80 mm après 800 impulsions |
| épaisseur d'ablation maximale = | 200,01 µm |

**EXEMPLE 7** (figure 12) : correction de la myopie :

| | |
|---|---|
| diamètre de la zone d'ablation : | 6,00 mm |
| diamètre du masque : | 10,00 mm |
| nombre d'anneaux : | 20 sur 1 zone |
| diamètre zone centrale : | 0,50 mm |
| surface occupée par les trous : | 49,58% |
| rayon de courbure initial = | 7,80 mm |
| rayon de courbure final = | 8,30 mm après 154 impulsions |
| épaisseur d'ablation maximale = | 38,62 µm |

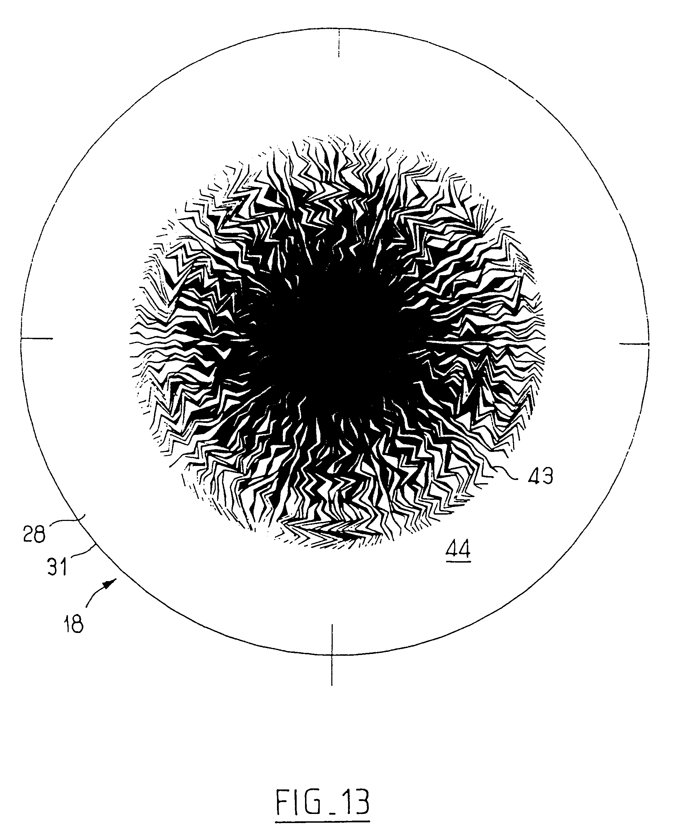

**EXEMPLE 8** (figure 13) : correction de la myopie

| | |
|---|---|
| diamètre de la zone d'ablation : | 6,00 mm |
| diamètre du masque : | 10,00 mm |
| nombre d'anneaux : | 20 sur 10 zones |
| diamètre zone centrale : | 0,50 mm |
| surface occupée par les trous : | 49,58% |
| rayon de courbure initial = | 7,80mm |
| rayon de courbure final = | 8,30 mm après 154 impulsions |
| épaisseur d'ablation maximale = | 38,62 µm |

**EXEMPLE 9** (figure 14) : correction de la myopie :

| | |
|---|---|
| diamètre de la zone d'ablation : | 6,00 mm |
| diamètre du masque : | 10,00 mm |
| nombre d'anneaux : | 20 sur 10 zones |
| Diamètre zone centrale : | 0,50 mm |
| surface occupée par les trous : | 49,58% |
| rayon de courbure initial = | 7,80 mm |
| rayon de courbure final = | 8,30 mm après 154 impulsions |
| épaisseur d'ablation maximale = | 38,62 µm |

Dans ces différents exemples, le nombre d'impulsions indiqué en relation avec le rayon de courbure final correspond à un tour complet du masque, dans un seul sens, autour de son axe 30 confondu avec l'axe 15, ou encore à un nombre entier de tels tours complets ou à des mouvements alternés de rotation autour de l'axe 30, effectués en synchronisme avec l'émission des impulsions dans des conditions telles que le masque occupe des positions angulaires différentes, autour de l'axe 30 lors des différentes impulsions.

Bien que chaque masque, notamment chacun des masques illustrés aux figures 6 à 14, soit conçu en fonction d'un rayon de courbure initial de la face antérieure 7 de la cornée 6, d'un rayon de courbure final de celle-ci et d'un nombre d'impulsions à appliquer pour obtenir ce rayon de courbure final à partir du rayon de courbure initial, un même masque peut être utilisé pour des valeurs différentes de ces trois paramètres, dont l'un peut être déterminé en fonction des deux autres au moyen d'abaques d'un type illustré à la figure 17 ou 18, ou, de préférence, au moyen d'un programme informatisé.

La figure 17 illustre, à titre d'exemple non limitatif, les différentes possibilités d'utilisation d'un masque prévu pour aboutir à un rayon de courbure de la face antérieure 7 de la cornée 6 de 8,4 mm, après 151 impulsions, à partir d'un rayon de courbure initial de 7,8 mm et permet de déterminer le nombre d'impulsions à utiliser pour aboutir à un rayon de courbure final déterminé à partir de sept rayons de courbure initiaux r₀.

Il en ressort, par exemple, qu'un rayon de courbure final de 8,8 mm pourrait être obtenu à partir d'un rayon de courbure initial r₀ de 7,8 mm après 240 impulsions et que ce même rayon de courbure final de 8,8 mm pourrait être obtenu après 90 impulsions pour un rayon de courbure initial r₀ de 8,4 mm.

La figure 18, illustre également à titre d'exemple non limitatif, les différentes possibilités d'utilisation d'un masque prévu pour aboutir à un rayon de courbure de la face antérieure 7 de la cornée 6 de 7,4 mm, après 100 impulsions, à partir d'un rayon de courbure initial de 7,8 mm et permet de déterminer le nombre d'impulsions à utiliser pour aboutir à un rayon de courbure final déterminé à partir de sept rayons de courbure initiaux r₀.

Ainsi, le nombre de masques différents qu'il est nécessaire de garder à disposition pour faire face à tous les types de correction peut être réduit à moins d'une dizaine, dont un critère de choix est le diamètre de la zone d'ablation.

Les masques qui viennent d'être décrits à titre d'exemple non limitatif, ou d'autres masques conformes à la présente invention, peuvent être utilisés non seulement dans une disposition selon laquelle leur axe 30 se confond avec l'axe 15, mais également dans une disposition selon laquelle leur axe 30 est sécant de l'axe 15 en un point 33 qui correspond à l'intersection de cet axe 30 avec le plan moyen 27, c'est-à-dire dans des conditions telles que ce plan moyen 27 ne soit plus perpendiculaire à l'axe 15 ; l'axe 30 autour duquel tourne le masque 18 forme alors par rapport à l'axe 15 un angle compris par exemple entre 50° et 62° comme le montre la figure 16, d'où il ressort que ces inclinaisons correspondent respectivement à la correction d'un astigmatisme de 1,5 dioptries et à la correction d'un astigmatisme de 3,5 dioptries, avec variation approximativement linéaire de l'angle à donner à l'axe 30 par rapport à l'axe 15 en fonction de l'astigmatisme à corriger, entre ces valeurs.

Ceci ne constitue toutefois que des exemples non limitatifs.

A cet effet, ainsi que pour permettre en outre la confusion de l'axe 30 avec l'axe 15 en vue d'une ablation sans correction d'astigmatisme, chaque masque 18 peut être indifféremment monté dans le "delivery system" S d'une façon schématisée à la figure 15 à laquelle on se réfèrera à présent.

Il doit être bien entendu que cette figure 15 correspond à un schéma de principe, par rapport auquel un Homme du métier pourra prévoir de nombreuses variantes d'exécution pratique.

Dans cet exemple schématique, le "delivery system" 5 est délimité, entre ses zones correspondant respectivement aux miroirs 14 et 19, c'est-à-dire dans la zone d'axe 15 du faisceau laser 3, par une enveloppe tubulaire 34 d'axe 15, parcourue intérieurement par le faisceau 3 et logeant intérieurement la lentille 16 et, dans la zone 17, le masque 18.

A cet effet, l'enveloppe tubulaire 34 présente intérieurement, dans la zone 17, des moyens 35, schématisés sous forme de deux couronnes annulaires de révolution autour de l'axe 15 et mutuellement espacées suivant cet axe, pour guider un bâti 36 à la rotation autour de l'axe 15 par rapport à l'enveloppe tubulaire 34, sans autre possibilité de déplacement relatif. Ce bâti 36 est également représenté sous la forme d'une couronne annulaire de révolution autour de l'axe 15, interposée entre les deux couronnes formant les moyens de guidage 35. Les moyens de guidage 35 comme le bâti 36 sont dimensionnés de façon à ne pas constituer d'obstacle au passage du faisceau 3, c'est-à-dire sont localisés à l'extérieur de celui-ci, à proximité immédiate de l'enveloppe tubulaire 34, au plus loin de l'axe 15.

Le bâti 36 porte lui-même, au pivotement relatif autour d'un axe 37 perpendiculaire à l'axe 15 et dont l'image se confond avec celle du point 33 à la figure 15, un support plat 38, annulaire de révolution autour d'un axe qui est sécant de l'axe 37, perpendiculairement à celui-ci, au point d'intersection de cet axe 37 avec l'axe 15 ; on comprendra ultérieurement que ce point d'intersection et cet axe du support 38 se confondent respectivement avec le point 33 et avec l'axe 30.

Le support 38 peut ainsi être réglé en orientation, par rapport au bâti 36, de telle sorte que son axe, à savoir l'axe 30, puisse soit se confondre avec l'axe 15, ce qui correspond à l'orientation du support 38 illustrée en trait plein à la figure 15, soit se décaler angulairement autour de l'axe 37 par rapport à cette orientation, dans un sens comme dans l'autre, de telle sorte que l'axe 30 prenne des orientations différentes de celles de l'axe 15 comme on l'a illustré en trait mixte en 30a et 30b, dans des limites correspondant à la formation entre l'axe 30 et l'axe 15 d'un angle α ou β, respectivement d'un côté ou de l'autre de l'axe 15, avec une valeur maximale au moins égale à 62° en application des indications données en référence à la figure 16.

De préférence, des moyens non illustrés, mais dont la réalisation relève des aptitudes normales d'un Homme du métier, sont prévus d'une part pour assurer un indexage de l'orientation du support 38 à l'intérieur du bâti 36, par exemple en termes d'angle α ou β formé par l'axe 30 en référence à l'axe 15, et d'autre part pour immobiliser le support 38 à l'encontre d'un pivotement autour de l'axe 37 par rapport au bâti 36 lorsqu'un réglage a été effectué.

Naturellement, les dimensions du support 38 sont telles que, même dans son orientation limite correspondant à l'angle α ou β maximal en référence à l'axe 15, il reste extérieur au faisceau 3, c'est-à-dire ne constitue pas d'obstacle au passage du faisceau 3.

Des moyens sont en outre prévus pour régler le bâti 36 en orientation, autour de l'axe 15, à l'intérieur de l'enveloppe tubulaire 34 et on a illustré à cet effet, à titre d'exemple non limitatif, un doigt de manoeuvre 39 solidaire du bâti 36 et traversant l'enveloppe 34 par une lumière 40 de celle-ci ; la lumière 40 s'étend sur au moins 90° autour de l'axe 15 lorsque l'axe 30 peut pivoter dans les deux sens autour de l'axe 37, par rapport à l'axe 15, comme il est illustré à la figure 15, et sur au moins 180° dans une variante non illustrée selon laquelle l'axe 30 peut pivoter d'un seul côté, autour de l'axe 37, par rapport à l'axe 15. Le doigt 39 et la lumière 40 sont avantageusement munis de moyens d'indexage, par exemple gradués en degré d'angle, pour permettre d'assurer un réglage précis de l'orientation du bâti 36 autour de l'axe 15 à l'intérieur de l'enveloppe 34, et comportent avantageusement des moyens pour retenir le bâti 36 dans l'orientation voulue par rapport à l'enveloppe tubulaire 34 ; la réalisation de tels moyens relève des aptitudes normales d'un Homme du métier et peut résulter d'une simple friction du doigt 39 à l'intérieur de la lumière 30, ou encore du bâti 36 lui-même à l'intérieur de l'enveloppe 34.

Par l'intermédiaire de moyens de guidage à la rotation relative autour de l'axe 30 sans autre possibilité de déplacement relatif, la réalisation de tels moyens relevant des aptitudes normales d'un Homme du métier, le support 38 porte une monture annulaire 41, de révolution autour de l'axe 30, pour un masque 18 dans des conditions telles que le plan moyen 27 de celui-ci inclue l'axe 37, c'est-à-dire notamment coupe l'axe 15 au point 33. Les dimensions de la monture 41 sont telles qu'elle reste extérieure au faisceau 3, c'est-à-dire ne constitue pas d'obstacle au passage du faisceau 3 même dans les orientations limites précitées de l'axe 30 par rapport à l'axe 15, correspondant pour la monture 41 et le plan moyen 27 du masque 18 à des positions schématisées en trait mixte à la figure 15, respectivement 41a, 41b et en 27a, 27b.

Le support 38 porte en outre de façon solidaire, également en dehors du faisceau 3 dans toute orientation de ce support 38 autour de l'axe 37 par rapport au bâti 36, un moteur 42 d'entraînement commandé de la monture 41 à la rotation autour de l'axe 30 par rapport au support 38 ; il est bien entendu que le masque 18, monté dans cette monture 41 et retenu périphériquement dans cette dernière, de façon solidaire mais amovible, par des moyens dont la réalisation relève des aptitudes normales d'un Homme du métier, se comporte comme un tout solidaire avec la monture 41 lors du fonctionnement du laser.

Un Homme du métier comprendra aisément que l'entrainement du masque 18 à la rotation autour de l'axe 30, par le moteur 42 synchronisé avec les impulsions de la source 1, permet d'assurer une sommation des ablations élémentaires résultant de chacune de ces impulsions, dans la face antérieure 6 de la cornée 7, sur un disque circulaire lorsque l'axe 30 coïncide avec l'axe 15, ce qui correspond à une correction de myopie ou d'hypermétropie sans astigmatisme ou encore à une ablation sans correction suivant le masque utilisé, alors qu'il se traduit par une ablation sur une surface de contour elliptique, dont le grand axe, correspondant à l'image de l'axe de pivotement 37 sur la face antérieure 6 de la cornée 7, est placé en coïncidence avec l'axe d'astigmatisme par un réglage approprié de l'orientation du bâti 36 autour de l'axe 15 par rapport à l'enveloppe tubulaire 34, lorsque l'axe 30 est décalé angulairement par rapport à l'axe 15, ce qui permet alors d'effectuer une correction d'astigmatisme, et plus précisément d'un degré d'astigmatisme réglable par réglage de l'orientation de l'axe 30 autour de l'axe 15.

Naturellement, les moyens de montage d'un masque 18 à l'intérieur du "delivery system" 5 qui viennent d'être décrits ne constituent qu'un minimum schématique et un Homme du métier pourra prévoir tout moyen de télécommande approprié, en fonction du type d'intervention à effectuer, de l'orientation de l'axe 30 par rapport à l'axe 15 et de l'orientation du bâti 36 autour de l'axe 15 par rapport à l'enveloppe tubulaire 34, de même qu'un Homme du métier pourra prévoir des moyens télécommandés assurant l'interchangeabilité de plusieurs masques destinés à des types différents d'intervention, de façon à présenter ces masques indifféremment de telle sorte que leur axe 30 coïncide avec l'axe 15 ou coupe celui-ci au point 33, par exemple en adoptant des moyens déjà connus pour présenter ainsi plusieurs masques de conception traditionnelle, en alternance.

Naturellement, on peut admettre certaines tolérances quant au centrage des masques selon l'invention, tels qu'ils ont été décrits à titre d'exemples non limitatifs en référence aux figures 6 à 14, par rapport à leur axe de rotation à l'intérieur du "delivery system", c'est-à-dire quant à la coïncidence de leur axe propre 30 avec cet axe de rotation tel qu'il est défini mécaniquement par la coopération de la monture 41 par rapport au support 38.

Toutefois, un excentrement excessif peut se traduire, dans le cas d'une application du masque selon l'invention à la correction d'une myopie, avec profondeur d'ablation maximale au centre de la cornée, ou à une ablation sans correction, c'est-à-dire avec une profondeur d'ablation constante, par une pointe ou un creux au centre de la cornée.

Pour éviter un tel défaut lorsque le respect de tolérances admissibles dans l'excentrement, par des moyens mécaniques, serait trop coûteux, on peut utiliser pour effectuer une ablation de profil déterminé non pas un masque unique, mais deux versions d'un même masque selon l'invention, que l'on interpose et fait tourner successivement ou alternativement dans le faisceau, dans les conditions précédemment décrites, pour réaliser l'ablation désirée.

A cet effet, par exemple, on prévoit en deux exemplaires le montage décrit en référence à la figure 15, sur des moyens connus d'un Homme du métier, non illustrés, pour placer soit l'un de ces exemplaires, soit l'autre dans la même position dans le "delivery system".

Les deux versions du masque différent par une zone centrale circulaire, centrée sur l'axe propre 30 du masque, laquelle zone centrale est transparente au rayonnement dans le cas de l'une des versions et opaque au rayonnement dans l'autre version.

Elles sont par ailleurs rigoureusement identiques, c'est-à-dire présentent la même répartition de zones transparentes qu'un masque destiné à être utilisé en exemplaire unique pour réaliser une ablation de même profil en étant soumis à un nombre d'impulsions déterminé, et chaque exemplaire du masque est soumis à un nombre d'impulsions correspondant par exemple à la moitié de ce nombre déterminé, de telle sorte que le nombre cumulé des impulsions auxquelles sont soumis les deux exemplaires du masque lors de l'intervention soit égal à ce nombre déterminé.

L'ablation obtenue présente ainsi le profil désiré si ce n'est que la conformation de la zone centrale des deux versions du masque entraîne une ablation de profondeur constante dans une zone centrale de la cornée, c'est-à-dire dans une zone délimitée sur celle-ci par un cercle centré au moins approximativement au centre de la cornée et présentant un diamètre, par exemple de l'ordre de 1µm, tel qu'il n'en résulte aucune perturbation sensible dans la vision. Le diamètre limite de ce cercle, à cette fin, détermine celui de la zone centrale, respectivement transparente ou opaque, des deux versions du masque.

On a fait apparaître cette modification du profil d'ablation en pointillés à la figure 1, dans le cas d'une application à la correction d'une myopie.

Les figures 19 et 20, où l'on retrouve les mêmes références numériques qu'aux figures 6 à 14 pour désigner les mêmes parties de masque, illustrent à titre d'exemple non limitatif les deux versions 18A, 18B d'un même masque destiné à être utilisé pour la correction d'une myopie.

On y a indiqué respectivement en 45A, 45B la zone centrale circulaire, respectivement transparente ou opaque au rayonnement mais de même diamètre, centrée sur l'axe propre non référencé de chaque version du masque.

Chaque version est par ailleurs du type décrit en référence aux figures 6 à 8, dont la zone transparente 43 présente la forme de surfaces circulaires sécantes ou non sécantes, avec les caractéristiques suivantes, indiquées dans le même ordre qu'à propos des masques illustrés aux figures 6 à 14, étant entendu que le nombre d'impulsions indiqué correspond au nombre cumulé des impulsions auxquelles sont exposés les deux masques.

| | |
|---|---|
| Diamètre de la zone d'ablation : | 6,00 mm |
| Diamètre du masque : | 8,96 mm |
| Nombre de trous : | 261 |
| ⌀ : 30,0 µm minimum | 2400,0 µm maximum |
| | écartement mutuel : 60,0 µm |
| Surface occupée par les trous : | 25,8 % |
| Rayon de courbure initial = | 7,80 mm (43,2 D) |
| Rayon de courbure final = | 8,60 mm (39,1 D) après 472 impulsions |
| Epaisseur d'ablation maximale = | 58,91µm tolérance : 1,00 % |

Il est bien entendu, toutefois, que ces indications ne constituent qu'un exemple non limitatif et que cette variante de réalisation de l'invention, mettant en oeuvre deux versions d'un même masque ne différant que par le fait qu'une zone centrale circulaire de l'un est opaque alors que la zone correspondante de l'autre est opaque, peut concerner des masques présentant toute répartition fractale voulue de sa zone transparente.

De façon générale, l'invention qui vient d'être décrite pourra connaître de nombreuses variantes, tant en ce qui concerne le dessin des zones transparentes sur les masques qu'en ce qui concerne le mode de montage de ces masques dans un "delivery system".

## Revendications

1. Masque (18) destiné à être interposé dans un faisceau laser (3) de longueur d'onde déterminée, avec possibilité de rotation autour d'un axe déterminé (4, 15, 9) dudit faisceau (3), afin d'appliquer au moyen de celui-ci un traitement déterminé, notamment d'ablation superficielle selon un profil déterminé autour de l'axe (4, 15, 9) du faisceau (3), à un organe (7) ou un objet sécant de cet axe (4, 15, 9) du faisceau (3), notamment une cornée (7) ou une lentille destinée à être accolée à la cornée ou insérée dans celle-ci, le masque (18) comportant à cet effet une zone (44) opaque à ladite longueur d'onde et une zone (43) transparente à celle-ci, présentant une forme et une position, par rapport audit axe propre (30), déterminées par une loi liant leur dimension angulaire, autour de celui-ci, à l'éloignement par rapport à celui-ci de telle sorte qu'il en résulte la formation par le faisceau (3), sur ledit organe (7) ou objet, d'une image de forme et de position, par rapport à l'axe (4, 15, 9) du faisceau (3), déterminées en fonction dudit traitement, notamment dudit profil d'ablation,
caractérisé en ce que la forme et la position, par rapport audit axe propre (30), de la zone transparente (43) sont déterminées de telle sorte que celle-ci forme un ensemble fractal répondant à ladite loi.

2. Masque selon la revendication 1, caractérisé en ce qu'il est constitué par une plaque ajourée.

3. Masque selon la revendication 1, caractérisé en ce qu'il est constitué par une lame transparente à ladite longueur d'onde, opacifiée localement à cette longueur d'onde par un traitement de surface.

4. Masque selon l'une quelconque des revendications 1 à 3, caractérisé en ce que la forme de la zone transparente (43) correspond sensiblement à une altération et/ou un fractionnement, suivant une direction circonférentielle en référence audit axe propre (30), d'une forme euclidienne répondant à ladite loi, avec préservation de la dimension angulaire de ladite forme euclidienne en référence audit axe propre (30), par cumul en cas de fractionnement, en fonction de l'éloignement par rapport audit axe propre (30).

5. Masque selon l'une quelconque des revendications 1 à 4, caractérisé en ce que la zone transparente (43) présente la forme de surfaces circulaires sécantes et/ou non sécantes (figures 6 à 8).

6. Masque selon l'une quelconque des revendications 1 à 4, caractérisé en ce que la zone transparente (43) présente une forme ramifiée (figures 12 à 14).

7. Masque selon l'une quelconque des revendications 1 à 4, caractérisé en ce que la zone transparente (43) présente la forme d'au moins une plage de contour irrégulier (figures 9 à 11).

8. Masque selon l'une quelconque des revendications 1 à 7, pour la correction de la myopie, caractérisé en ce que la dimension angulaire, cumulée en cas de fractionnement, de la zone transparente (43), en référence à son axe propre (30), est maximale à proximité de celui-ci (figures 6, 9, 12 à 14).

9. Masque selon l'une quelconque des revendications 1 à 7, pour la correction de l'hypermétropie, caractérisé en ce que la dimension angulaire, cumulée en cas de fractionnement, de la zone transparente (43), en référence à son axe propre (30), est maximale à proximité de la périphérie (figures 7, 10).

10. Masque selon l'une quelconque des revendications 1 à 7, pour une ablation de profondeur constante, caractérisé en ce que la dimension angulaire, cumulée en cas de fractionnement, de la zone transparente (43), en référence à son axe propre (30), est sensiblement constante de cet axe (30) à la périphérie (figures 8, 11).

11. Procédé pour appliquer, au moyen d'un faisceau laser (3) de longueur d'onde déterminée et d'axe déterminé (4, 15, 9), un traitement déterminé, notamment d'ablation superficielle selon un profil déterminé autour dudit axe (4, 15, 9), à un organe (7) ou un objet sécant dudit axe (4, 15, 9), notamment une cornée (7) ou une lentille destinée à être accolée à la cornée ou insérée dans celle-ci, ledit procédé consistant notamment à interposer un masque (18) dans le faisceau (3) et à le faire tourner autour de l'axe (4, 15, 9) de celui-ci, par rapport à celui-ci,
caractérisé en ce que l'on utilise un masque (18) selon l'une quelconque des revendications 1 à 10, présentant un plan moyen (27) et un axe propre (30) perpendiculaire à celui-ci, on oriente cet axe propre (30) du masque (18) par rapport à l'axe (4, 15, 9) du faisceau (3) de telle sorte qu'ils coïncident, notamment pour corriger une myopie ou une hypermétropie sans astigmatisme ou pour effectuer une ablation de profondeur constante, ou qu'ils forment entre eux un angle prédéterminé (α, β), autour d'un axe de pivotement relatif (37) perpendiculaire à l'un et l'autre, notamment pour corriger un astigmatisme déterminé en faisant coïncider une image de l'axe de pivotement (37) sur la cornée (7) avec un axe d'astigmatisme, et on fait tourner le masque (18) autour de l'axe (4, 15, 9) du faisceau (3), par rapport à celui-ci, par rotation du masque (18) autour de son axe propre (27) par rapport au faisceau (3), pendant l'émission de celui-ci.

12. Procédé selon la revendication 11, caractérisé en ce que l'on fait tourner le masque (18) d'un tour, autour de son axe propre (30), par rapport au faisceau (3), pendant l'émission de celui-ci.

13. Procédé selon la revendication 11, caractérisé en ce que l'on fait tourner le masque (18) en alternance dans un sens et dans le sens opposé, notamment de façon aléatoire, et/ou sur plus d'un tour autour de son axe propre (30), par rapport au faisceau (3) pendant l'émission de celui-ci.

14. Procédé selon la revendication 13, caractérisé en ce que l'on émet le faisceau (3) par impulsions et en ce que l'on synchronise la rotation du masque (18) autour de son axe propre (30), par rapport au faisceau (3), et lesdites impulsions de telle sorte que le masque (18) occupe des positions angulaires différentes, autour de son axe propre (30), lors des différentes impulsions.

15. Dispositif pour appliquer, au moyen d'un faisceau laser (3) de longueur d'onde déterminée et d'axe déterminé (4, 15, 9), un traitement déterminé, notamment d'ablation superficielle selon un profil déterminé autour dudit axe (4, 15, 9), à un organe (7) ou un objet sécant dudit axe (4, 15, 9), notamment une cornée (7) ou une lentille destinée à être accolée à la cornée ou insérée dans celle-ci, ledit dispositif comportant notamment des moyens (36, 38, 41, 42) d'interposition d'un masque (18) dans le faisceau (3) avec possibilité de rotation autour de l'axe (4, 15, 9) de celui-ci, par rapport à celui-ci,
caractérisé en ce que, en vue de l'utilisation d'un masque (18) selon l'une quelconque des revendications 1 à 10, présentant un plan moyen (27) et un axe propre (30) perpendiculaire à celui-ci, les moyens d'interposition (36, 38, 41, 42) comportent:
- un bâti (36) extérieur au faisceau (3), monté réglable en orientation par rapport au faisceau (3), autour de l'axe (4, 15, 9) de celui-ci,
- un support (38) extérieur au faisceau (3), monté réglable en orientation par rapport au bâti (36), autour d'un axe de pivotement (37) perpendiculaire à l'axe (4, 15, 9) du faisceau (3),
- une monture (41) extérieure au faisceau (3), montée mobile en rotation sur le support (38), autour d'un axe de rotation (30) perpendiculaire à l'axe de pivotement (37) au même point (33) que l'axe (4, 15, 9) du faisceau (3), et susceptible de porter solidairement le masque (18) dans une position dans laquelle l'axe (30) de celui-ci coïncide avec l'axe de rotation (30),
- des moyens (42) pour entraîner la monture (41) en rotation par rapport au support (38), autour de l'axe de rotation (30), pendant l'émission du faisceau laser (3).

16. Dispositif selon la revendication 15, caractérisé en ce que la rotation de la monture (41) par rapport au support (38), autour de l'axe de rotation (30), s'effectue sur un tour.

17. Dispositif selon la revendication 15, caractérisé en ce que les moyens (42) pour entraîner la monture (41) en rotation sont propres à provoquer une rotation sur plus d'un tour et/ou en alternance dans des sens mutuellement opposés, notamment de façon aléatoire.

18. Dispositif selon la revendication 17, caractérisé en ce que le faisceau laser (3) est émis par impulsions et en ce que les moyens pour entraîner la monture (41) en rotation sont synchronisés avec les impulsions de telle sorte que la monture (41) occupe des positions angulaires différentes par rapport au support (38), autour de l'axe de rotation (30), lors des différentes impulsions.
